# EUROPEAN PATENT APPLICATION

(11) **EP 4 471 066 A1**
(43) Date of publication of application: **04.12.2024**
(21) Application number: 22922274.0
(22) Date of filing: 24.01.2022
(51) Int. Cl.: C07K 16/30, C07K 16/28, A61P 35/00, A61K 39/00

(54) **ANTIBODY SPECIFICALLY BINDING TO MUC-1 AND USES THEREOF**

(71) Applicant: Cyron Therapeutics Co., Ltd., Daegu 43023 (KR)
(72) Inventor: MOON, Yoo Ri, Seoul 05351 (KR); YOON, Sangsoon, Seoul 05236 (KR); JO, Tae Yeon, Seoul 05337 (KR); KIM, Ah Young, Ansan-si, Gyeonggi-do 15575 (KR); YU, So Dam, Hanam-si, Gyeonggi-do 12913 (KR)
(74) Representative: J A Kemp LLP
(86) International application number: PCT/KR2022/001255
(87) International publication number: WO 2023/140412

(57) **Abstract**

Provided are an anti-MUC1 antibody, an anti-MUC1/anti-CD3 bispecific antibody, and a pharmaceutical composition comprising same for prevention and/or treatment of cancer.

## Description

### [TECHNICAL FIELD]

Provided are an anti-MUC1 antibody, an anti-MUC1/anti-CD3 bispecific antibody, and a pharmaceutical composition comprising same for prevention and/or treatment of cancer.

### [BACKGROUND ART]

MUC1 (mucin-1) is one kind of mucin glycoproteins, and is a transmembrane glycoprotein comprising multiple glycosylated extracellular domains. The MUC1 length is 200 to 500 nm on the cell surface, and MUC1 is located in the apical membrane of normal epithelial cells. MUC1 is expressed in glandular or luminal epithelial cells of many organs such as breast, stomach, esophagus, pancreas, urethra, lung, kidney and gallbladder. Mucin is overexpressed in many cancer types, including cancers of pancreas, lung, breast, ovary, and the like. Many studies have been conducted on the pathological relationship between mucin and cancer, and in particular, several antibodies against MUC1 are known in the art.

However, their therapeutic efficacy can still be improved, and accordingly, the present application provides an anti-MUC1 antibody or an antigen binding fragment thereof with improved characteristics.

### [DISCLOSURE]

### [TECHNICAL PROBLEM]

One aspect provides an anti-MUC1 antibody or an antigen binding fragment thereof.

Another aspect provides an anti-MUC1/anti-CD3 bispecific antibody, comprising the anti-MUC1 antibody or antigen binding fragment thereof, and anti-CD3 antibody or antigen binding fragment thereof.

Other aspect provides a pharmaceutical composition for prevention or treatment of cancer, comprising (i) the anti-MUC1 antibody or antigen binding fragment thereof, or (ii) the anti-MUC1/anti-CD3 bispecific antibody as an active ingredient.

Other aspect provides a method for prevention or treatment of cancer, comprising administering (i) the anti-MUC1 antibody or antigen binding fragment thereof, or (ii) the anti-MUC1/anti-CD3 bispecific antibody into a subject in need of prevention or treatment of cancer.

Other aspect provides a use of (i) the anti-MUC1 antibody or antigen binding fragment thereof, or (ii) the anti-MUC1/anti-CD3 bispecific antibody for using in prevention or treatment of cancer, or a use thereof for using in preparation of an anti-cancer agent.

Other aspect provides a polynucleotide encoding (i) the anti-MUC1 antibody or antigen binding fragment thereof, or (ii) the anti-MUC1/anti-CD3 bispecific antibody, a recombinant vector comprising the polynucleotide, and/or a recombinant cell comprising the recombinant vector.

Other aspect provides a method for preparation of (i) the anti-MUC1 antibody or antigen binding fragment thereof, or (ii) the anti-MUC1/anti-CD3 bispecific antibody, comprising administering expressing a polynucleotide encoding (i) the anti-MUC1 antibody or antigen binding fragment thereof, or (ii) the anti-MUC1/anti-CD3 bispecific antibody.

### [TECHNICAL SOLUTION]

### Definition of terms

When an animal-derived antibody produced by immunizing an immunized animal with a desired antigen is administered to a human on a therapeutic purpose, immunorejection may be generally caused, and in order to inhibit this immunorejection, a chimeric antibody has been developed. The chimeric antibody is one in which the constant region of an animal-derived antibody, which causes an anti-isotype response, is substituted with the constant region of a human antibody. The chimeric antibody has significantly improved the anti-isotype response compared to the animal-derived antibody, but animal-derived amino acids are still present in the variable region, and therefore, potential side effects on an anti-idiotypic response are contained. What was developed to improve these side effects is a humanized antibody. This is manufactured by grafting CDR (complementarity determining regions) regions, which play an important role in binding of an antigen among variable regions of the chimeric antibody into a human antibody framework.

In one specific embodiment, the anti-MUC1 antibody may be an animal-derived antibody (for example, mouse-derived antibody), a chimeric antibody (for example, mouse-human chimeric antibody), or a humanized antibody. The antibody or antigen binding fragment may be isolated from a living body or non-naturally occurring. The antibody or antigen binding fragment may be recombinantly or synthetically produced. The antibody or antigen binding fragment may be isolated from a living body or non-naturally occurring. The antibody or antigen binding fragment may be synthetically or recombinantly produced.

In another embodiment, the antibody may be derived (isolated) from any animal including mammals including humans, birds, and the like. For example, the antibody may be an antibody of a human, mouse, donkey, sheep, rabbit, goat, guinea pig, camel, horse or chicken. Herein, the human antibody is an antibody having an amino acid sequence of a human immunoglobulin, and includes an antibody isolated from a human immunoglobulin library or an antibody isolated from an animal transfected against at least one human immunoglobulin and not expressing intrinsic immunoglobulins.

The anti-MUC1 antibody may be a monoclonal antibody or polyclonal antibody, and for example, it may be a monoclonal antibody. The monoclonal antibody may be manufactured according to a method widely known in the art. For example, it may be manufactured using the phage display method. Otherwise, the anti-MUC1 antibody may be prepared as a mouse-derived monoclonal antibody.

In the anti-MUC1 antibody or antigen binding fragment thereof, the other sites except for heavy chain CDR and light chain CDR regions, or heavy chain variable regions and light chain variable regions may be derived from all subtypes of immunoglobulins (for example, IgA, IgD, IgE, IgG (IgG1, IgG2, IgG3, IgG4), IgM, etc.), and for example, they may be derived from framework sites, and/or light chain variable regions and/or heavy chain variable regions of the all subtypes of immunoglobulins.

A complete antibody (for example, IgG type) has a structure having 2 full length light chains and 2 full length heavy chains, and each light chain is linked to the heavy chain by a disulfide bond. Variable regions of the antibody are divided into heavy chain variable regions and light chain variable regions, and the heavy chain variable regions have gamma ( γ ), mu ( µ ), alpha ( α ), delta ( δ ) and epsilon ( ε ) types, and have gamma1 ( γ 1), gamma2 ( γ 2), gamma3 ( γ 3), gamma4 ( γ 4), alpha1 ( α 1) and alpha2 ( α 2) as subclasses. The light chain variable regions have kappa ( κ ) and lambda ( λ ) types.

The term, "heavy chain" is interpreted as a meaning which includes all of a full-length heavy chain comprising a variable region domain V_{H} comprising an amino acid sequence having a variable region sequence sufficient to give specificity to an antigen, and 3 constant region domains, C_{H1}, C_{H2} and C_{H3} and hinge, and fragments thereof. In addition, the term, "light chain" is interpreted as a meaning which includes all of a full-length light chain comprising a variable region domain V_{L} comprising an amino acid sequence having a variable region sequence sufficient to give specificity to an antigen, and a constant region domain, C_{L}, and fragments thereof.

The term, "CDR (complementarity determining region)" refers to an amino acid sequence of hypervariable regions of a heavy chain and a light chain of an immunoglobulin. The heavy chain and light chain may comprise 3 CDRs each (CDRH1, CDRH2, CDRH3 and CDRL1, CDRL2, CDRL3). The CDR may provide a major contact residue for binding an antibody to an antigen or epitope. On the other hand, in the present description, the term, "specifically bind" or "specifically recognize" is same as the meaning commonly known in the art, and refers to that an antigen and an antibody specifically interact to conduct an immunological reaction.

The term, "hinge region" is a region comprised in a heavy chain of an antibody, and is present between CH1 and CH2 regions, and means a region functioning to provide flexibility of an antigen binding site in the antibody.

When an animal-derived antibody goes through a chimerization process, the animal-derived IgG1 hinge is substituted with a human IgG1 hinge, but the animal-derived IgG1 hinge has a shorter length than the human IgG1 hinge, and the disulfide bonds between two heavy chains were reduced from 3 to 2, and thereby, effects of the rigidity of the hinge different from each other are shown. Therefore, modification of the hinge region can increase antigen binding efficiency of a humanized antibody. Methods for deleting, adding or substituting amino acids to modify the amino acid sequence of the hinge region are well known in the art.

The "antigen binding fragment" is a fragment of the entire structure of an immunoglobulin, and means a part of a polypeptide comprising a portion to which an antigen can bind. For example, it may be scFv, (scFv)₂, scFvFc, Fab, Fab' or F(ab')₂, but not limited thereto. In the present invention, the antigen binding fragment of the antibody may be an antigen fragment comprising at least one complementarity determining region, and for example, it may be selected from the group consisting of scFv, (scFv)2, scFv-Fc, Fab, Fab' and F(ab')2. The antigen binding fragment may be obtained using proteinase (for example, Fab can be obtained by restriction cutting of the whole antibody with papain, and F(ab')₂ fragment can be obtained by cutting it with pepsin), and may be manufactured through genetic recombination technology.

Fab of the antigen binding fragment has a structure with variable regions of light chains and heavy chains, and constant regions of light chains and the first constant region (C_{H1}) of the heavy chain, and has one antigen binding site.

Fab' is different from Fab in that it has a hinge region comprising at least one cysteine residue the C-terminus of the heavy chain C_{H1} domain. F(ab')₂ antibody is produced as cysteine residues in the hinge region form a disulfide bond.

Fv is a minimal antibody fragment having only the heavy chain variable region and light chain variable region, and a recombinant technology that produces the Fv fragment is well known in the art. In the two-chain Fv, the heavy chain variable region and light chain variable region are linked by a non-covalent bond, and in the single-chain Fv (scFv), generally, through a peptide linker, the heavy chain variable region and single-chain variable region are linked by a covalent bond or linked right at the C-terminus, and thus, it may form a structure as a dimer as same as the two-chain Fv. The peptide linker may be same as described above, and for example, it may be in length of 1 to 100 amino acids, for example, in length of 2 to 50 amino acids, or in length of 5 to 25 amino acids, and there is no limit to the type of the amino acids comprised therein.

The antigen binding fragment may be obtained using protease (for example, Fab can be obtained by restriction cutting of the whole antibody with papain, and F(ab')₂ fragment can be obtained by cutting it with pepsin), and may be manufactured through genetic recombination technology.

In the antigen binding fragment, for example, scFV, the heavy chain variable region and light chain variable region may be linked through or not through a linker, for example, a peptide linker. The peptide linker may be a polypeptide consisting of any amino acids of 1 to 100 or 2 to 50, and there is no limit to the type of the amino acids comprised therein. The peptide linker may comprise for example, Gly, Asn and/or Ser residues, and may also comprise neutral amino acids such as Thr and/or Ala. The length of the linker may be variously determined, as long as it does not affect the function of the bispecific antibody. For example, the peptide linker may be comprised by comprising at least one amino acid selected from the group consisting of Gly, Asn, Ser, Thr and Ala of 1 to 100, 2 to 50, or 5 to 25 in total. In one embodiment, the peptide linker may be expressed as (G4S)n (n is a repetition number of (G4S), an integer of 1 to 10, for example, an integer of 2 to 5).

The term, "antagonist" is interpreted to a concept of including all molecules that partially or completely block, inhibit, or neutralize at least one biological activity of a target (for example, MUC1). For example, the "antagonist" antibody refers to an antibody that inhibits or reduces biological activity of an antigen to which the antibody binds (for example, MUC1). The antagonist may act to reduce receptor phosphorylation, or incapacitating or killing cells activated by a ligand, by binding to a receptor for a ligand (target). In addition, the antagonist may completely disrupt the receptor-ligand interaction, bind to the receptor competitively with the ligand, or substantially reduce the receptor-ligand interaction by changing or down regulation of the tertiary structure of the receptor.

### Anti-MUC1 antibody or antigen binding fragment thereof

One embodiment of the present invention provides a polypeptide which can specifically recognize and/or specifically bind to MUC1 (Mucin-1).

The MUC1 may be human MUC1, or MUC1 of a monkey, dog, rat, or mouse, or the like. In one embodiment, the human MUC1 may be MUC1 isoform M2 (identifier: A6ZID5; NCBI reference number: ABS01294.1), MUC1 isoform 1 (identifier: P15941-1), MUC1 isoform 2 (identifier: P15941-2), MUC1 isoform 3 (identifier: P15941-3), MUC1 isoform 4 (identifier: P15941-4), MUC1 isoform 5 (identifier: P15941-5), MUC1 isoform 6 (identifier: P15941-6), MUC1 isoform Y (identifier: P15941-7), MUC1 isoform 8 (identifier: P15941-8), MUC1 isoform 9 (identifier: P15941-9), MUC1 isoform F (identifier: P15941-10), MUC1 isoform Y-LSP (identifier: P15941-11), MUC1 isoform S2 (identifier: P15941-12), MUC1 isoform M6 (identifier: P15941-13), MUC1 isoform ZD (identifier: P15941-14), MUC1 isoform T10 (identifier: P15941-15), MUC1 isoform E2 (identifier: P15941-16), or MUC1 isoform J13 (identifier: P15941-17), or the like, but not limited thereto.

In one embodiment, the human MUC1 may have an amino acid sequence provided in NCBI Reference Sequence: NP_001018016.1, NP_001018017.1, NP_001037855.1, NP_001037856.1, NP_001037857.1, NP_001037858.1, NP_001191214.1, NP_001191215.1, NP_001191216.1, NP_001191217.1, NP_001191218.1, NP_001191219.1, NP_001191220.1, NP_001191221.1, NP_001191222.1, NP_001191223.1, NP_001191224.1, NP_001191225.1, NP_001191226.1, NP_002447.4, or the like, but not limited thereto, and each amino acid sequence may be encoded by a nucleotide sequence (mRNA) provided in NCBI Reference Sequence: NM_001018016.2, NM_001018017.2, NM_001044390.2, NM_001044391.2, NM_001044392.2, NM_001044393.2, NM_001204285.1, NM_001204286.1, NM_001204287.1, NM_001204288.1, NM_001204289.1, NM_001204290.1, NM_001204291.1, NM_001204292.1, NM_001204293.1, NM_001204294.1, NM_001204295.1, NM_001204296.1, NM_001204297.1, NM_002456.5 or the like, but not limited thereto.

In the present description, that a protein, polypeptide or polynucleotide has an amino acid sequence or nucleotide sequence may be used to mean all the cases in which the protein, polypeptide or polynucleotide comprises the sequence, or consists essentially of the sequence, or consist of the sequence.

These polypeptides may be used as complementarity determining regions (CDRs) of the anti-MUC1 antibody. Then, the complementarity determining regions may be determined by all common methods, and for example, they may be determined by IMGT definition (http://www.imgt.org/IMGT_vquest/share/textes/) or Kabat definition (http://www.bioinf.org.uk/abs/), but not limited thereto.

In other embodiment, provided is an MUC1 target polypeptide molecule comprising at least one selected from the group consisting of the polypeptides.

The MUC1 target polypeptide molecule may comprise heavy chain complementarity determining regions and light chain complementarity determining regions of the afore-mentioned anti-MUC1 antibody, or a combination thereof; or heavy chain variable regions comprising the heavy chain complementarity determining regions, light chain variable regions comprising the light chain complementarity determining regions, or a combination thereof.

The MUC1 target polypeptide molecule, is not limited thereto, but may play a role as the anti-MUC1 antibody, an antigen binding fragment of the antibody, or an analogue of the anti-MUC1 antibody (structure having the backbone and function similar to the antibody; for example, peptibody, nanobody, etc.), or a precursor or component part (for example, CDR) such as a multiple binding antibody and the like.

The term, "peptibody (peptide + antibody)" is a fusion protein in which all or part of constant regions such as Fc region and the like of the peptide and antibody, and refers to a protein having the backbone and function similar to the antibody. Herein, the afore-mentioned at least one peptide may act as the antigen binding site (heavy chain and/or light chain CDR or variable regions).

The term, "nanobody" is also called single-domain antibody, and refers to an antibody fragment comprising the single variable domain of the antibody in a monomer form, and has a characteristic of selectively binding to a specific antigen similarly to an antibody in a complete structure. The molecular weight of the nanobody is generally about 12 kDa to about 15 kDa, and it is very small compared to a general molecular weight (about 150 kDa to about 160 kDa) of the complete antibody (comprising two heavy chains and two light chains), and in some cases, it is smaller than Fab fragment or scFv fragment.

Specifically, the present invention provides a polypeptide which can specifically recognize and/or specifically bind to MUC1. The polypeptide may comprise at least one amino acid sequence selected from the group consisting of SEQ ID NO: 1, SEQ ID NO: 7, SEQ ID NO: 16, SEQ ID NO: 19, SEQ ID NO: 27, and SEQ ID NO: 33, and in more detail, it may have at least one amino acid sequence selected from the group consisting of SEQ ID NO: 2 to 6, SEQ ID NO: 8 to 15, SEQ ID NO: 17 to 18, SEQ ID NO: 20 to 26, SEQ ID NO: 28 to 32, and SEQ ID NO: 34 and SEQ ID NO: 38.

The polypeptide and their applicable complementarity determining regions are summarized in Table 1 below. In Table 1 below, V_{H}-CDR1, V_{H}-CDR2, and V_{H}-CDR3 refer to heavy chain complementarity determining regions, and V_{L}-CDR1, V_{L}-CDR2, and V_{L}-CDR3 refer to light chain complementarity determining regions.

**[Table 1]**

| Name | Sequence | SEQ ID NO: |
|---|---|---|
| VH-CDR1 | **X1**-Y-W-M-**X2** (X1=D or S; X2=N, S, or H) | 1 |
| VH-CDR1 | DYWMN | 2 |
| VH-CDR1 | DYWM**S** | 3 |
| VH-CDR1 | **S**YWMN | 4 |
| VH-CDR1 | **S**YWM**S** | 5 |
| VH-CDR1 | **S**YWM**H** | 6 |
| VH-CDR2 | X3-I-X4-X5-K-X6-Y-X7-X8-X9-X10-X11-Y-X12-X13-S-V-K- G (X3=Q, F, or V; X4=R or K; X5=N, S, Q, or Y; X6=P or A; X7=N or G; X8=Y, G, or S; X9=E or T; X10=T or K; X11=Y or E; X12=S, A, orV;X13=N, Q, or A) | 7 |
| VH-CDR2 | QIRNKPYNYETYYSNSVKG | 8 |
| VH-CDR2 | QIRNKPYNYETYYS**Q**SVKG | 9 |
| VH-CDR2 | QIR**S**KPYNYETYY**AA**SVKG | 10 |
| VH-CDR2 | **F**IR**S**K**A**Y**GGT**T**E**Y**AA**SVKG | 11 |
| VH-CDR2 | QI**KQ**KPYNYE**K**YY**VQ**SVKG | 12 |
| VH-CDR2 | QI**KQ**KPY**GG**E**K**YY**VQ**SVKG | 13 |
| VH-CDR2 | QIRNKPYNYE**K**YY**AQ**SVKG | 14 |
| VH-CDR2 | **V**IR**Y**KPY**GS**E**K**YY**AQ**SVKG | 15 |
| VH-CDR3 | A-M-I-T-S-D-G-Y-A-**X14**-D-Y (X14=M or F) | 16 |
| VH-CDR3 | AMITSDGYAMDY | 17 |
| VH-CDR3 | AMITSDGYA**F**DY | 18 |
| VL-CDR1 | **X15**-**X16**-S-Q-S-**X17**-L-**X18**-S-S-**X19-X20-X21**-N-Y-L-A (X15=K or R; X16=S or A; X17=L or V; X18=L, Y, or H; X19=N or S; X20=Q or G; X21=K or Y) | 19 |
| VL-CDR1 | KSSQSLLLSSNQKNYLA | 20 |
| VL-CDR1 | KSSQS**V**LLSSNQKNYLA | 21 |
| VL-CDR1 | KSSQS**V**L**Y**SSNQKNYLA | 22 |
| VL-CDR1 | **R**SSQSLLLSSNQKNYLA | 23 |
| VL-CDR1 | **RA**SQS**V**LLSSNQKNYLA | 24 |
| VL-CDR1 | **R**ASQS**V**LLSS**S**QKNYLA | 25 |
| VL-CDR1 | **R**SSQSLL**H**SSN**GY**NYLA | 26 |
| VL-CDR2 | W-**X22**-S-**X23**-R-**X24**-**X25** (X22=A or G; X23=T or S; X24=E or A; X25=S or T) | 27 |
| VL-CDR2 | WASTRES | 28 |
| VL-CDR2 | WAS**S**RE**T** | 29 |
| VL-CDR2 | WAS**S**R**AT** | 30 |
| VL-CDR2 | W**G**STRES | 31 |
| VL-CDR2 | W**G**STR**A**S | 32 |
| VL-CDR3 | Q-Q-**X26**-F-**X27**-**X28**-P-**X29**-T (X26=C or S; X27=S or Q; X28=Y, T, or S; X29=L or Y) | 33 |
| VL-CDR3 | QQCFSYPLT | 34 |
| VL-CDR3 | QQ**S**FSYPLT | 35 |
| VL-CDR3 | QQ**S**FS**T**P**Y**T | 36 |
| VL-CDR3 | QQ**S**FS**S**P**Y**T | 37 |
| VL-CDR3 | QQ**S**F**QT**P**Y**T | 38 |

In one embodiment, provided is an anti-MUC1 antibody or antigen binding fragment thereof comprising at least one selected from the group consisting of the polypeptides as complementarity determining regions.

The anti-MUC1 antibody or antigen binding fragment thereof may comprise at least one selected from the group consisting of
a polypeptide having the amino acid sequence of SEQ ID NO: 1 (VH-CDR1),
a polypeptide having the amino acid sequence of SEQ ID NO: 7 (VH-CDR2), and
a polypeptide having the amino acid sequence of SEQ ID NO: 16 (VH-CDR3),
as heavy chain complementarity determining regions.

The amino acid sequence of SEQ ID NO: 1 is same as the amino acid sequence of the following General formula 1:

[General formula 1] (SEQ ID NO: 1) **X1**-Y-W-M-**X2**

X1 is D or S,
X2 is N, S, or H.

In one embodiment, the polypeptide of SEQ ID NO: 1 which can be used as light chain CDR1 of the anti-MUC1 antibody or antigen binding fragment thereof, may have at least one amino acid sequence selected from the group consisting of, for example, SEQ ID NOs: 2 to 6, and the detailed sequences are described in Table 1 above and sequence listing, and the boldly underlined parts in the described amino acid sequence represent modified amino acids.

The amino acid sequence of SEQ ID NO: 7 is same as the amino acid sequence of the following General formula 2:

[General formula 2] (SEQ ID NO: 7) **X3**-I-**X4**-**X5**-K-**X6**-Y-**X7**-**X8**-**X9**-**X10**-**X11**-Y-**X12**-**X13**-S-V-K-G

X3 is Q, F, or V,
X4 is R or K,
X5 is N, S, Q, orY,
X6 is P or A,
X7 is N or G,
X8 is Y, G, or S,
X9 is E or T,
X10 is T or K,
X11 is Y or E,
X12 is S, A, or V,
X13 is N, Q, or A.

In one embodiment, the polypeptide of SEQ ID NO: 7 which can be used as heavy chain CDR2 of the anti-MUC1 antibody or antigen binding fragment thereof, may have at least one amino acid sequence selected from the group consisting of, for example, SEQ ID NOs: 8 to 15, and the detailed sequences are described in Table 1 above and sequence listing, and the boldly underlined parts in the described amino acid sequence represent modified amino acids.

The amino acid sequence of SEQ ID NO: 16 is same as the amino acid sequence of the following General formula 3:

[General formula 3] (SEQ ID NO: 16) A-M-I-T-S-D-G-Y A-**X14**-D-Y

X14 is M or F.

In one embodiment, the polypeptide of SEQ ID NO: 16 which can be used as heavy chain CDR3 of the anti-MUC1 antibody or antigen binding fragment thereof, may have at least one amino acid sequence selected from the group consisting of, for example, SEQ ID NOs: 17 to 18, and the detailed sequences are described in Table 1 above and sequence listing, and the boldly underlined parts in the described amino acid sequence represent modified amino acids.

The anti-MUC1 antibody or antigen binding fragment thereof may comprise at least one selected from the group consisting of
a polypeptide having the amino acid sequence of SEQ ID NO: 19 (VL-CDR1),
a polypeptide having the amino acid sequence of SEQ ID NO: 27 (VL-CDR2), and
a polypeptide having the amino acid sequence of SEQ ID NO: 33 (VL-CDR3),
as light chain complementarity determining regions.

The amino acid sequence of SEQ ID NO: 19 is same as the amino acid sequence of the following General formula 4:

[General formula 4] (SEQ ID NO: 19) **X15**-**X16**-S-Q-S-**X17**-L-**X18**-S-S**-X19-X20-X21**-N-Y-L-A

X15 is KorR,
X16 is S or A,
X17 is L or V,
X18 is L, Y, or H,
X19 is N or S,
X20 is Q or G,
X21 is K or Y

In one embodiment, the polypeptide of SEQ ID NO: 19 which can be used as light chain CDR1 of the anti-MUC1 antibody or antigen binding fragment thereof, may have at least one amino acid sequence selected from the group consisting of, for example, SEQ ID NOs: 20 to 26, and the detailed sequences are described in Table 1 above and sequence listing, and the boldly underlined parts in the described amino acid sequence represent modified amino acids.

The amino acid sequence of SEQ ID NO: 27 is same as the amino acid sequence of the following General formula 5:

[General formula 5] (SEQ ID NO: 27) W**-X22**-S-**X23**-R-**X24-X25**

X22 is A or G,
X23 is TorS,
X24 is E or A,
X25 is S or T.

In one embodiment, the polypeptide of SEQ ID NO: 27 which can be used as light chain CDR2 of the anti-MUC1 antibody or antigen binding fragment thereof may have at least one amino acid sequence selected from the group consisting of, for example, SEQ ID NOs: 28 to 32, and the detailed sequences are described in Table 1 above and sequence listing, and the boldly underlined parts in the described amino acid sequence represent modified amino acids.

The amino acid sequence of SEQ ID NO: 33 is same as the amino acid sequence of the following General formula 6:

[General formula 6] (SEQ ID NO: 33) Q-Q-**X26**-F-**X27**-**X28**-P-**X29**-T

X26 is C or S,
X27 is S or Q,
X28 is Y, T, or S,
X29 is L or Y

In one embodiment, the polypeptide of SEQ ID NO: 27 which can be used as light chain CDR3 of the anti-MUC1 antibody or antigen binding fragment thereof, may have at least one amino acid sequence selected from the group consisting of, for example, SEQ ID NOs: 34 to 38, and the detailed sequences are described in Table 1 above and sequence listing, and the boldly underlined parts in the described amino acid sequence represent modified amino acids.

In one embodiment, the anti-MUC1 antibody or antigen binding fragment thereof may comprise
at least one heavy chain complementarity determining region selected from the group consisting of a polypeptide having the amino acid sequence of SEQ ID NO: 1 (VH-CDR1), a polypeptide having the amino acid sequence of SEQ ID NO: 7 (VH-CDR2), and a polypeptide having the amino acid sequence of SEQ ID NO: 16 (VH-CDR3), or a heavy chain variable region comprising the at least one heavy chain complementarity determining region;
at least one light chain complementarity determining region selected from the group consisting of a polypeptide having the amino acid sequence of SEQ ID NO: 19 (VL-CDR1), a polypeptide having the amino acid sequence of SEQ ID NO: 27 (VL-CDR2), and a polypeptide having the amino acid sequence of SEQ ID NO: 33 (VL-CDR3), or a light chain variable region comprising the at least one light chain complementarity determining region;
a combination of the heavy chain complementarity determining region and light chain complementarity determining region; or
a combination of the heavy chain variable region and light chain variable region.

In one embodiment, the anti-MUC1 antibody or antigen binding fragment thereof may comprise
at least one heavy chain complementarity determining region selected from the group consisting of a polypeptide having an amino acid sequence of SEQ ID NOs: 2 to 6 (VH-CDR1), a polypeptide having an amino acid sequence of SEQ ID NOs: 8 to 16 (VH-CDR2), and a polypeptide having an amino acid sequence of SEQ ID NOs: 17 to 18 (VH-CDR3), or a heavy chain variable region comprising the at least one heavy chain complementarity determining region;
at least one light chain complementarity determining region selected from the group consisting of a polypeptide having an amino acid sequence of SEQ ID NOs: 20 to 26 (VL-CDR1), a polypeptide having an amino acid sequence of SEQ ID NOs: 28 to 32 (VL-CDR2), and a polypeptide having an amino acid sequence of SEQ ID NOs: 34 to 38 (VL-CDR3), or a light chain variable region comprising the at least one light chain complementarity determining region;
a combination of the heavy chain complementarity determining region and light chain complementarity determining region; or
a combination of the heavy chain variable region and light chain variable region.

In one embodiment, the anti-MUC1 antibody or antigen binding fragment thereof may comprise
a heavy chain variable region comprising a polypeptide having an amino acid sequence of SEQ ID NOs: 2 to 6 (VH-CDR1), a polypeptide having an amino acid sequence of SEQ ID NOs: 8 to 16 (VH-CDR2), and a polypeptide having an amino acid sequence of SEQ ID NOs: 17 to 18 (VH-CDR3); and
a light chain variable region comprising a polypeptide having an amino acid sequence of SEQ ID NOs: 20 to 26 (VL-CDR1), a polypeptide having an amino acid sequence of SEQ ID NOs: 28 to 32 (VL-CDR2), and a polypeptide having an amino acid sequence of SEQ ID NOs: 34 to 38 (VL-CDR3).

In one embodiment, the anti-MUC1 antibody or antigen binding fragment thereof may comprise CDR sequences of the heavy chain variable regions and CDR sequences of the light chain variable regions combined in Table 2 and Table 3 below.

**[Table 2]**

| Name of heavy chain variable region | VH-CDR1 | SE Q ID NO: | VH-CDR2 | SE Q ID NO: | VH-CDR3 | SE Q ID NO: |
|---|---|---|---|---|---|---|
| VH0: VH-chimera | DYWMN | 2 | QIRNKPYNYETY YSNSVKG | 8 | AMITSDGYAM DY | 17 |
| VH1 | DYWMN | 2 | QIRNKPYNYETY YSQSVKG | 9 | AMITSDGYAM DY | 17 |
| VH2 | DYWMN | 2 | QIRSKPYNYETY YAASVKG | 10 | AMITSDGYAM DY | 17 |
| VH3 | DYWMS | 3 | FIRSKAYGGTTE YAASVKG | 11 | AMITSDGYAFD Y | 18 |
| VH4 | DYWMN | 2 | QIRNKPYNYETY YSNSVKG | 8 | AMITSDGYAM DY | 17 |
| VH5 | SYWMN | 4 | QIKQKPYNYEKY YVQSVKG | 12 | AMITSDGYAM DY | 17 |
| VH6 | SYWMS | 5 | QIKQKPYGGEK YYVQSVKG | 13 | AMITSDGYAFD Y | 18 |
| VH7 | DYWMN | 2 | QIRNKPYNYETY YSNSVKG | 8 | AMITSDGYAM DY | 17 |
| VH8 | SYWMN | 4 | QIRNKPYNYEKY YAQSVKG | 14 | AMITSDGYAM DY | 17 |
| VH9 | SYWMH | 6 | VIRYKPYGSEKY YAQSVKG | 15 | AMITSDGYAFD Y | 18 |

**[Table 3]**

| Name of light chain variable region | VL-CDR1 | SE Q ID NO: | VL-CDR2 | SE Q ID NO: | VL-CDR3 | SE Q ID NO: |
|---|---|---|---|---|---|---|
| VL0: VL-chimera | KSSQSLL LSSNQKN YLA | 20 | WASTRES | 28 | QQCFSYPLT | 34 |
| VL1 | KSSQSLL LSSNQKN YLA | 20 | WASTRES | 28 | QQCFSYPLT | 34 |
| VL2 | KSSQSVL LSSNQKN YLA | 21 | WASTRES | 28 | QQSFSYPLT | 35 |
| VL3 | KSSQSVL YSSNQKN YLA | 22 | WASTRES | 28 | QQSFSTPYT | 36 |
| VL4 | RSSQSLL LSSNQKN YLA | 23 | WASTRES | 28 | QQCFSYPLT | 34 |
| VL5 | RASQSVL LSSNQKN YLA | 24 | WASSRET | 29 | QQSFSYPLT | 35 |
| VL6 | RASQSVL LSSSQKN YLA | 25 | WASSRAT | 30 | QQSFSSPYT | 37 |
| VL7 | RSSQSLL LSSNQKN YLA | 23 | WASTRES | 28 | QQCFSYPLT | 34 |
| VL8 | RSSQSLL LSSNQKN YLA | 23 | WGSTRES | 31 | QQSFSYPLT | 35 |
| VL9 | RSSQSLL HSSNGYN YLA | 26 | WGSTRAS | 32 | QQSFQTPYT | 38 |

In one embodiment, the anti-MUC1 antibody or antigen binding fragment thereof may comprise
at least one heavy chain complementarity determining region selected from the group consisting of a polypeptide having an amino acid sequence of SEQ ID NO: 2 or SEQ ID NO: 4 (VH-CDR1), a polypeptide having an amino acid sequence of SEQ ID NO: 8, SEQ ID NO: 9, or SEQ ID NO: 14 (VH-CDR2), and a polypeptide having the amino acid sequence of SEQ ID NO: 17 (VH-CDR3), or a heavy chain variable region comprising the at least one heavy chain complementarity determining region;
at least one light chain complementarity determining region selected from the group consisting of a polypeptide having an amino acid sequence of SEQ ID NO: 21, SEQ ID NO: 22, SEQ ID NO: 23, or SEQ ID NO: 24 (VL-CDR1), a polypeptide having an amino acid sequence of SEQ ID NO: 28, SEQ ID NO: 29, or SEQ ID NO: 31 (VL-CDR2), and a polypeptide having an amino acid sequence of SEQ ID NO: 34, SEQ ID NO: 35, or SEQ ID NO: 36 (VL-CDR3), or a light chain variable region comprising the at least one light chain complementarity determining region;
a combination of the heavy chain complementarity determining region and light chain complementarity determining region; or
a combination of the heavy chain variable region and light chain variable region.

In one embodiment, the anti-MUC1 antibody or antigen binding fragment thereof may comprise
at least one heavy chain complementarity determining region selected from the group consisting of a polypeptide having the amino acid sequence of SEQ ID NO: 2 (VH-CDR1), a polypeptide having an amino acid sequence of SEQ ID NO: 8 or SEQ ID NO: 9 (VH-CDR2), and a polypeptide having the amino acid sequence of SEQ ID NO: 17 (VH-CDR3), or a heavy chain variable region comprising the at least one heavy chain complementarity determining region;
at least one light chain complementarity determining region selected from the group consisting of a polypeptide having an amino acid sequence of SEQ ID NO: 21, SEQ ID NO: 23, or SEQ ID NO: 24 (VL-CDR1), a polypeptide having an amino acid sequence of SEQ ID NO: 28, or SEQ ID NO: 29 (VL-CDR2), and a polypeptide having an amino acid sequence of SEQ ID NO: 34 or SEQ ID NO: 35 (VL-CDR3), or a light chain variable region comprising the at least one light chain complementarity determining region;
a combination of the heavy chain complementarity determining region and light chain complementarity determining region; or
a combination of the heavy chain variable region and light chain variable region.

In one embodiment, the anti-MUC1 antibody or antigen binding fragment thereof
may comprise
1) a polypeptide having the amino acid sequence of SEQ ID NO: 2 (VH-CDR1), a polypeptide having the amino acid sequence of SEQ ID NO: 9 (VH-CDR2), a polypeptide having the amino acid sequence of SEQ ID NO: 17 (VH-CDR3), a polypeptide having the amino acid sequence of SEQ ID NO: 21 (VL-CDR1), a polypeptide having the amino acid sequence of SEQ ID NO: 28 (VL-CDR2), and a polypeptide having the amino acid sequence of SEQ ID NO: 35 (VL-CDR3);
2) a polypeptide having the amino acid sequence of SEQ ID NO: 2 (VH-CDR1), a polypeptide having the amino acid sequence of SEQ ID NO: 9 (VH-CDR2), a polypeptide having the amino acid sequence of SEQ ID NO: 17 (VH-CDR3), a polypeptide having the amino acid sequence of SEQ ID NO: 23 (VL-CDR1), a polypeptide having the amino acid sequence of SEQ ID NO: 28 (VL-CDR2), and a polypeptide having the amino acid sequence of SEQ ID NO: 36 (VL-CDR3);
3) a polypeptide having the amino acid sequence of SEQ ID NO: 2 (VH-CDR1), a polypeptide having the amino acid sequence of SEQ ID NO: 9 (VH-CDR2), a polypeptide having the amino acid sequence of SEQ ID NO: 17 (VH-CDR3), a polypeptide having the amino acid sequence of SEQ ID NO: 24 (VL-CDR1), a polypeptide having the amino acid sequence of SEQ ID NO: 29 (VL-CDR2), and a polypeptide having the amino acid sequence of SEQ ID NO: 35 (VL-CDR3);
4) a polypeptide having the amino acid sequence of SEQ ID NO: 2 (VH-CDR1), a polypeptide having the amino acid sequence of SEQ ID NO: 8 (VH-CDR2), a polypeptide having the amino acid sequence of SEQ ID NO: 17 (VH-CDR3), a polypeptide having the amino acid sequence of SEQ ID NO: 23 (VL-CDR1), a polypeptide having the amino acid sequence of SEQ ID NO: 28 (VL-CDR2), and a polypeptide having the amino acid sequence of SEQ ID NO: 34 (VL-CDR3); or
5) a polypeptide having the amino acid sequence of SEQ ID NO: 2 (VH-CDR1), a polypeptide having the amino acid sequence of SEQ ID NO: 8 (VH-CDR2), a polypeptide having the amino acid sequence of SEQ ID NO: 17 (VH-CDR3), a polypeptide having the amino acid sequence of SEQ ID NO: 24 (VL-CDR1), a polypeptide having the amino acid sequence of SEQ ID NO: 29 (VL-CDR2), and a polypeptide having the amino acid sequence of SEQ ID NO: 35 (VL-CDR3).

The anti-MUC1 antibody or antigen binding fragment thereof may comprise a framework sequence of at least one heavy chain variable region, a framework sequence of at least one light chain variable region, or a framework sequence of at least one heavy chain variable region and a framework sequence of at least one light chain variable region. One example of the framework sequence of the light chain variable region and the framework sequence of the light chain variable region is shown in Table 4 to Table 7 below.

**[Table 4]**

| Framework of heavy chain variable region | FR1 | SEQ ID NO: | FR2 | SEQ ID NO: |
|---|---|---|---|---|
| VH0: VH-chimera | EVKVDETGGGLVQPGRP MKLSCVVSGITFS | 39 | WVRQSPEKGLEWVA | 49 |
| VH1 | EVKVVESGGGLVQPGRS LRLSCTASGITFS | 40 | WVRQAPGKGLEWVG | 50 |
| VH2 | EVQLVESGGGLVQPGRS LRLSCTASGITFG | 41 | WVRQAPGKGLEWVG | 50 |
| VH3 | EVQLVESGGGLVQPGRS LRLSCTASGFTFG | 42 | WVRQAPGKGLEWVG | 50 |
| VH4 | EVKVVESGGGLVQPGGS LRLSCAASGITFS | 43 | WVRQAPGKGLEWVA | 51 |
| VH5 | EVQLVESGGGLVQPGGS LRLSCAASGITFS | 44 | WVRQAPGKGLEWVA | 51 |
| VH6 | EVQLVESGGGLVQPGGS LRLSCAASGFTFS | 45 | WVRQAPGKGLEWVA | 51 |
| VH7 | QVKVVESGGGVVQPGR SLRLSCAASGITFS | 46 | WVRQAPGKGLEWVA | 51 |
| VH8 | QVQLVESGGGVVQPGRS LRLSCAASGITFS | 47 | WVRQAPGKGLEWVA | 51 |
| VH9 | QVQLVESGGGVVQPGRS LRLSCAASGFTFS | 48 | WVRQAPGKGLEWVA | 51 |

**[Table 5]**

| Framework of heavy chain variable region | FR3 | SE Q ID NO: | FR4 | SE Q ID NO: |
|---|---|---|---|---|
| VH0: VH-chimera | RFTISRDDSKSSVYLQMN NLRAEDMGIYYCTW | 52 | WGQGTSVTVSS | 58 |
| VH1 | RFTISRDDSKSIAYLQMSSL KTEDTAVYYCTW | 53 | WGQGTLVTVSS | 59 |
| VH2 | RFTISRDDSKSIAYLQMSSL KTEDTAVYYCTW | 53 | WGQGTLVTVSS | 59 |
| VH3 | RFTISRDDSKSIAYLQMSSL | 53 | WGQGTLVTVSS | 59 |
| | KTEDTAVYYCTW | | | |
| VH4 | RFTISRDDAKSSVYLQMSS LRAEDTAVYYCTW | 54 | WGQGTLVTVSS | 59 |
| VH5 | RFTISRDDAKSSLYLQMSS LRAEDTAVYYCAW | 55 | WGQGTLVTVSS | 59 |
| VH6 | RFTISRDDAKSSLYLQMSS LRAEDTAVYYCAW | 55 | WGQGTLVTVSS | 59 |
| VH7 | RFTISRDDSKSTVYLQMSS LRAEDTAVYYCTW | 56 | WGQGTLVTVSS | 59 |
| VH8 | RFTISRDDSKSTLYLQMSS LRAEDTAVYYCAW | 57 | WGQGTLVTVSS | 59 |
| VH9 | RFTISRDDSKSTLYLQMSS LRAEDTAVYYCAW | 57 | WGQGTLVTVSS | 59 |

**[Table 6]**

| Framework of light chain variable region | FR1 | SE QID NO: | FR2 | SEQ ID NO: |
|---|---|---|---|---|
| VL0: VL-chimera | DIVMSQSPSSLAVSVGEKV TLIC | 60 | WYQQKPGQSPKLLlY | 65 |
| VL1 | DIVMTQSPDSLAVSLGERA TISC | 61 | WYQQKPGQPPKLLIY | 66 |
| VL2 | DIVMTQSPDSLAVSLGERA TISC | 61 | WYQQKPGQPPKLLIY | 66 |
| VL3 | DIVMTQSPDSLAVSLGERA TISC | 61 | WYQQKPGQPPKLLIY | 66 |
| VL4 | EIVMTQSPGTLSLSPGERA TLSC | 62 | WYQQKPGQAPRLLIY | 67 |
| VL5 | EIVLTQSPGTLSLSPGERA TLSC | 63 | WYQQKPGQAPRLLIY | 67 |
| VL6 | EIVLTQSPGTLSLSPGERA TLSC | 63 | WYQQKPGQAPRLLIY | 67 |
| VL7 | DIVMTQSPLSLPVTPGEPA SISC | 64 | WYLQKPGQSPQLLlY | 68 |
| VL8 | DIVMTQSPLSLPVTPGEPA SISC | 64 | WYLQKPGQSPQLLlY | 68 |
| VL9 | DIVMTQSPLSLPVTPGEPA SISC | 64 | WYLQKPGQSPQLLIY | 68 |

**Table 7]**

| Framework of light chain variable region | FR3 | SEQ ID NO: | FR4 | SEQ ID NO: |
|---|---|---|---|---|
| VL0: VL-chimera | GVPDRFTGSGSGTDFT LTINSVKAEDLAVYYC | 69 | FGAGTKLELK | 75 |
| VL1 | GVPDRFSGSGSGTEFT LTISSVQAEDVAVYYC | 70 | FGQGTKLEIK | 76 |
| VL2 | GVPDRFSGSGSGTEFT LTISSLQAEDVAVYYC | 71 | FGQGTKLEIK | 76 |
| VL3 | GVPDRFSGSGSGTEFT LTISSLQAEDVAVYYC | 71 | FGQGTKLEIK | 76 |
| VL4 | GIPDRFSGSGSGTDFTL TISRVEPEDFAVYYC | 72 | FGQGTKLEIK | 76 |
| VL5 | GIPDRFSGSGSGTDFTL TISRLEPEDFAVYYC | 73 | FGQGTKLEIK | 76 |
| VL6 | GIPDRFSGSGSGTDFTL TISRLEPEDFAVYYC | 73 | FGQGTKLEIK | 76 |
| VL7 | GVPDRFSGSGSGTDFT LKISRVEAEDVGVYYC | 74 | FGQGTKLEIK | 76 |
| VL8 | VPDRFSGSGSGTDFTL KISRVEAEDVGVYYC | 74 | FGQGTKLEIK | 76 |
| VL9 | GVPDRFSGSGSGTDFT LKISRVEAEDVGVYYC | 74 | FGQGTKLEIK | 76 |

In one embodiment, the anti-MUC1 antibody or antigen binding fragment thereof
may be a heavy chain variable region comprising the heavy chain complementarity determining region (VH0 to VH9 in Table 2 above), a light chain variable region comprising the light chain complementarity determining region (VL0 to VL9 in Table 3 above), or a combination thereof. For example, the heavy chain variable region may comprise an amino acid sequence selected from the group consisting of SEQ ID NOs: 77 to 86, and the light chain variable region may comprise an amino acid sequence selected from the group consisting of SEQ ID NOs: 87 to 96.

Accordingly, the anti-MUC1 antibody or antigen binding fragment thereof
may comprise a heavy chain variable region having an amino acid sequence selected from the group consisting of SEQ ID NOs: 77 to 86;
a light chain variable region having an amino acid sequence selected from the group consisting of SEQ ID NOs: 87 to 96; or
a combination thereof.

In one embodiment, the anti-MUC1 antibody or antigen binding fragment thereof
may comprise a heavy chain variable region having an amino acid sequence of SEQ ID NO: 78, SEQ ID NO: 81, SEQ ID NO: 84, or SEQ ID NO: 85; and
a light chain variable region having an amino acid sequence of SEQ ID NO: 89, SEQ ID NO: 90, SEQ ID NO: 91, SEQ ID NO: 92, SEQ ID NO: 94, or SEQ ID NO: 95.

In one embodiment, the anti-MUC1 antibody or antigen binding fragment thereof
may comprise a heavy chain variable region having an amino acid sequence of SEQ ID NO: 78, SEQ ID NO: 81, or SEQ ID NO: 84; and
a light chain variable region having an amino acid sequence of SEQ ID NO: 89, SEQ ID NO: 91, or SEQ ID NO: 92.

In one embodiment, the anti-MUC1 antibody or antigen binding fragment thereof
may comprise
1) a heavy chain variable region having the amino acid sequence of SEQ ID NO: 78 and a light chain variable region having the amino acid sequence of SEQ ID NO: 89;
2) a heavy chain variable region having the amino acid sequence of SEQ ID NO: 78 and a light chain variable region having the amino acid sequence of SEQ ID NO: 91;
3) a heavy chain variable region having the amino acid sequence of SEQ ID NO: 78 and a light chain variable region having the amino acid sequence of SEQ ID NO: 92;
4) a heavy chain variable region having the amino acid sequence of SEQ ID NO: 81 and a light chain variable region having the amino acid sequence of SEQ ID NO: 91;
5) a heavy chain variable region having the amino acid sequence of SEQ ID NO: 81 and a light chain variable region having the amino acid sequence of SEQ ID NO: 92; or
6) a heavy chain variable region having the amino acid sequence of SEQ ID NO: 84 and a light chain variable region having the amino acid sequence of SEQ ID NO: 91.

The term, "epitope" is interpreted to mean an antigenic determinant, a portion of an antigen recognized by an antibody. In one specific embodiment, the epitope may be a polypeptide comprising a region within the MUC1 protein, for example, the amino acid sequence of SEQ ID NO: 109.

Therefore, the anti-MUC1 antibody or antigen binding fragment may specifically bind to an epitope comprising the amino acid sequence of SEQ ID NO: 109.

### Multiple binding antibody

One embodiment of the present invention relates to a multiple binding antibody or multispecific antibody comprising a polypeptide which can specifically recognize and/or specifically bind to MUC1, and more specifically, it may be a bispecific antibody comprising a polypeptide which can specifically recognize and/or specifically bind to a second target, together with a polypeptide which can specifically recognize and/or specifically bind to MUC1.

In the present description, the term, "multiple binding antibody" refers to an antibody that recognizes and/or binds to 2 or more different antigens, or recognizes and/or binds to sites different from each other of the same antigen, and one antigen binding site of the multiple binding antibody may comprise the polypeptide described above.

Other one embodiment of the present invention relates to a bispecific antigen-binding molecule comprising a first antigen-binding domain specifically binding to MUC1 and a second antigen-binding domain specifically binding to CD3. Specifically, one embodiment of the present invention, relates to a bispecific antibody, comprising at least one antigen binding site specific to the MUC1 antibody or antigen binding fragment thereof according to the present invention and a T cell activating antigen.

In the bispecific antibody, the T cell activating antigen may be a CD3 T cell co-receptor (CD3; cluster of differentiation 3) antigen (for example, anti-CD3 epsilon (ε) antigen), and the bispecific antibody may comprise an anti-CD3 antibody comprising an antigen binding site against a CD3 antigen or an antigen binding fragment thereof.

Specifically, it relates to a bispecific antibody comprising the anti-MUC1 antibody or antigen binding fragment thereof, and the anti-CD3 antibody or antigen binding fragment thereof (in other words, anti-MUC1/anti-CD3 bispecific antibody). The anti-MUC1/anti-CD3 bispecific antibody may exhibit a synergistic effect while maintaining the affinity and activity to each antigen, MUC1 and CD3.

The bispecific antibody specifically binds to the first antigen, MUC1, and may comprise the anti-MUC1 antibody or antigen binding fragment thereof. The anti-MUC1 antibody or antigen binding fragment thereof may be same as described above.

In addition, the bispecific antibody specifically binds to the second antigen, CD3, and specifically, it may comprise the anti-CD3 antibody or antigen binding fragment thereof. The anti-Cd3 antibody or antigen binding fragment may be an antibody or antigen binding fragment thereof, which binds to CD3 of a human, monkey, mouse, or the like. The anti-CD3 antibody or antigen binding fragment thereof is useful for targeting T cells expressing CD3, or stimulating T cell activation. The anti-CD3 antibody or antigen binding fragment thereof of the present invention may be comprised as a part of the bispecific antibody that directs CD3-mediated T cell activation against specific cell types, for example, tumor cells or infectious agents.

The anti-CD3 antibody or antigen binding fragment thereof may be used without limitation as long as it is an antibody specifically binding to CD3, and for example, an OKT3 antibody or antigen binding fragment thereof, a UCHT-1 antibody or antigen binding fragment thereof, an SP34 antibody or antigen binding fragment thereof, or the like, but not limited thereto. The OKT3 antibody may be a mouse or humanized immunoglobulin IgG2a isotype monoclonal antibody. The UCHT-1 antibody is known to react with a ~20kDa CD3ε small unit of a human T cell receptor (TCR)/CD3 complex, and the SP34 antibody is known to bind to a CD3ε small unit. The method for acquiring the OKT3 antibody, UCHT-1 antibody, or SP34 antibody is commonly well known in the art.

In one specific embodiment, the anti-CD3 antibody or antigen binding fragment thereof may comprise at least one CDR sequence, preferably, 6 CDR sequences, selected from the group consisting of the following polypeptides (See Table 8 below):
a polypeptide having the amino acid sequence of SEQ ID NO: 97 (VH-CDR1),
a polypeptide having the amino acid sequence of SEQ ID NO: 98 (VH-CDR2),
a polypeptide having the amino acid sequence of SEQ ID NO: 99 (VH-CDR3),
a polypeptide having the amino acid sequence of SEQ ID NO: 100 (VL-CDR1),
a polypeptide having the amino acid sequence of SEQ ID NO: 101 (VL-CDR2), and
a polypeptide having the amino acid sequence of SEQ ID NO: 102 (VL-CDR3). More specifically, the anti-CD3 antibody or antigen binding fragment thereof
may comprise a heavy chain variable region having the amino acid sequence of SEQ ID NO: 103, and a light chain variable region having the amino acid sequence of SEQ ID NO: 104 or SEQ ID NO: 105.

**[Table 8]**

| CD3 antibody | Sequence | SEQ ID NO: |
|---|---|---|
| VH-CDR1 | GYTFTRYT | 97 |
| VH-CDR2 | INPSRGYT | 98 |
| VH-CDR3 | ARYYDDHYSLDY | 99 |
| VL-CDR1 | ASSSVSY | 100 |
| VL-CDR2 | DTS | 101 |
| VL-CDR3 | QQWSSNPFT | 102 |
| VH | QVQLVQSGGGVVQPGRSLRLSCKASGYTFTRYTMHW VRQAPGKGLEWIGYINPSRGYTNYNQKVKDRFTISRD NSKNTAFLQMDSLRPEDTGVYFCARYYDDHYSLDYW GQGTPVTVSS | 103 |
| VL | | 104 |

The antigen binding fragment of the anti-CD3 antibody may be selected from the group consisting of scFv, (scFv)2, Fab, Fab' and F(ab')2 of the anti-CD3 antibody.

The anti-MUC1/anti-CD3 bispecific antibody according to the present invention may be one in which the anti-CD3 antibody or antigen binding fragment thereof is linked to the C-terminus or N-terminus of the anti-MUC1 antibody or antigen binding fragment thereof.

For example, it may be the anti-CD3 antibody scFv combined to the heavy chain N-terminus or C-terminus of the anti-MUC1 antibody or antigen binding fragment thereof (anti-MUC1 heavy chain N-term-anti-CD3 scFv or anti-MUC1 heavy chain C-term-anti-CD3 scFv), or anti-CD3 antibody scFv combined to the light chain N-terminus or C-terminus of the anti-MUC1 (anti-MUC1 light chain N-term-anti-CD3 scFv or anti-MUC1 light chain C-term-anti-CD3 scFv). In addition, it may be a bispecific antibody in a form of having one antigen binding site of the anti-MUC1 antibody and one antigen binding site of the anti-CD3 antibody (1+1), having two and one each, 3 antigen binding sites in one bispecific antibody (1+2 or 2+1), or having 4, two each, antigen binding sites (2+2), including a bispecific antibody in which the anti-CD3 scFv is combined in a monovalent or divalent form, but not limited thereto.

In one embodiment, the anti-MUC1/anti-CD3 bispecific antibody may comprise the anti-MUC1 antibody or antigen binding fragment thereof, and the anti-CD3 antibody or antigen binding fragment thereof linked to the C-terminus or N-terminus of the heavy chain or light chain, for example, the N-terminus of the light chain of the anti-MUC1 antibody or antigen binding fragment thereof. In one embodiment, the anti-MUC1/anti-CD3 bispecific antibody may comprise a complete antibody against MUC1 (for example, IgG type antibody) and an antigen binding fragment (for example, scFv, (scFv)2, scFv-Fc, Fab, Fab' and F(ab')2) of the anti-CD3 antibody linked to the N-terminus of the light chain of the antibody.

In the anti-MUC1/anti-CD3 bispecific antibody, the anti-MUC1 antibody or antigen binding fragment thereof and the anti-CD3 antibody or antigen binding fragment thereof may be linked through or not through a linker, for example, a peptide linker. In addition, the heavy chain part and light chain part in the antigen binding fragment, for example, the heavy chain variable region and light chain variable region in the scFv fragment may be also linked through or not through a peptide linker. The peptide linker connecting the anti-MUC1 antibody or antigen binding fragment and the anti-CD3 antibody or antigen binding fragment thereof, and the peptide linker connecting the heavy chain part and light chain part in the antigen binding fragment may be same or different. The peptide linker may be a polypeptide consisting of any amino acids of 1 to 100, or 2 to 50, and there is no limit to the type of the amino acids comprised therein. The peptide linker may comprise for example, Gly, Asn and/or Ser residues, and may also comprise neutral amino acids such as Thr and/or Ala. The amino acid sequence suitable for the peptide linker is known in the art. On the other hand, the length of the linker may be variously determined, as long as it does not affect the function of the bispecific antibody. For example, the peptide linker may be composed of at least one amino acid sequence selected from the group consisting of Gly, Asn, Ser, Thr and Ala of 1 to 100, 2 to 50, or 5 to 25 in total. In one embodiment, the peptide linker may be expressed as (G4S)n (n is a repetition number of (G4S), an integer of 1 to 10, for example, an integer of 2 to 5).

In one embodiment, the anti-MUC1/anti-CD3 bispecific antibody may be in a form of scFv of the anti-CD3 antibody combined to the N-terminus of the light chain positioned in the anti-MUC1 antibody or antigen binding fragment thereof. The anti-MUC1/anti-CD3 bispecific antibody in which the anti-CD3 antibody scFv is combined to the N-terminus of the light chain positioned in the anti-MUC1 antibody or antigen binding fragment thereof as above may have more excellent cytotoxicity against cancer cells, cancer cell proliferation inhibiting effects, and/or cancer treatment effects, than the anti-MUC1/anti-CD3 bispecific antibody in which the anti-CD3 antibody scFv is combined to the C-terminus of the light chain positioned in the anti-MUC1 antibody or antigen binding fragment thereof.

### Medical use

Other aspect provides a pharmaceutical composition for preventing or treating cancer, comprising the anti-MUC1 antibody or antigen binding fragment, or the anti-MUC1/anti-CD3 bispecific antibody or antigen binding fragment.

Other aspect provides a method for preventing or treating cancer, comprising administering a pharmaceutically effective dose of the anti-MUC1 antibody or antigen binding fragment, or the anti-MUC1/anti-CD3 bispecific antibody or antigen binding fragment into a subject in need of prevention or treatment of cancer. The method for preventing and/or treating cancer may further comprise confirming a patient in need of prevention and/or treatment of cancer before the administering.

The anti-MUC1 antibody or antigen binding fragment, or the anti-MUC1/anti-CD3 bispecific antibody or antigen binding fragment may be usefully used for prevention or treatment of cancer. The anti-MUC1 antibody or antigen binding fragment, or the anti-MUC1/anti-CD3 bispecific antibody or antigen binding fragment provided in the present description exhibits a function as an agonist of MUC1, and therefore, in order that the anti-MUC1 antibody or antigen binding fragment, or the anti-MUC1/anti-CD3 bispecific antibody or antigen binding fragment exhibits efficacy (for example, anti-cancer efficacy such as cancer cell death and the like), and for the cancer to be applied, it may be advantageous for the corresponding cancer cells to express MUC1, and for example, it may be solid cancer expressing MUC1. Specifically, one embodiment of the present invention relates to a novel antibody specifically binding to MUC-1 with improved solid cancer targeting and treatment and a use thereof. In one embodiment, the cancer may include at least one selected from the group consisting of blood cancer (for example, B cell lymphoma, etc.), osteomyelitis, thyroid cancer, uterine cancer (for example, cervical cancer), skin cancer, melanoma, breast cancer, colorectal cancer, lung cancer, gastric cancer, prostate cancer and pancreatic cancer, or solid cancer in which the epitope of the antigen to which the anti-MUC1 antibody or antigen binding fragment specifically binds is expressed by 30% or more, but not limited thereto.

In the pharmaceutical composition or method, the pharmaceutically effective dose of the anti-MUC1 antibody or antigen binding fragment thereof, or the anti-MUC1/anti-CD3 bispecific antibody or antigen binding fragment thereof may be provided with at least one additive selected from the group consisting of pharmaceutically acceptable carriers, diluents, and excipients and the like.

The pharmaceutically acceptable carriers are commonly used for preparation of an antibody, and may be at least one selected from the group consisting of lactose, dextrose, sucrose, sorbitol, mannitol, starch, acacia gum, calcium phosphate, alginate, gelatin, calcium silicate, microcrystalline cellulose, polyvinyl pyrrolidone, cellulose, water, syrup, methyl cellulose, methylhydroxybenzoate, propylhydroxybenzoate, talc, magnesium stearate, mineral oil, and the like, but not limited thereto. The pharmaceutical composition may further comprise at least one selected from the group consisting of diluents, excipients, lubricants, wetting agents, sweeteners, flavoring agents, emulsifiers, suspending agents, preservatives, and the like commonly used for preparing a pharmaceutical composition in addition to the above components.

The pharmaceutical composition, the anti-MUC1 antibody or antigen binding fragment thereof, or the anti-MUC1/anti-CD3 bispecific antibody or antigen binding fragment may be orally or parenterally administered. In case of parenteral administration, it may be administered by intravenous injection, subcutaneous injection, intramuscular injection, intraperitoneal injection, intradermal administration, topical administration, intranasal administration, intrapulmonary administration and intrarectal administration and the like. In case of oral administration, protein or peptide is digested, so an oral composition should be formulated to coat an active drug or protect it from degradation in the stomach. In addition, the composition may be administered with any device in which an active substance can move to a target cell.

An appropriate dosage of the pharmaceutical composition, the anti-MUC1 antibody or antigen binding fragment, or the anti-MUC1/anti-CD3 bispecific antibody or antigen binding fragment thereof may be variously prescribed by factors such as the preparation method, administration method, a patient's age, body weight, gender, morbid condition, food, administration time, administration route, excretion rate, and reaction sensitivity. For example, a daily dosage of the pharmaceutical composition, the anti-MUC1 antibody or antigen binding fragment, or the anti-MUC1/anti-CD3 bispecific antibody or antigen binding fragment thereof may be in a range of 0.001 to 1000mg/kg, specifically, 0.01 to 100mg/kg, more specifically, 0.1 to 50 mg/kg, but not limited thereto. The daily dosage may be formulated as one preparation in a unit dosage form, or be formulated by appropriately distributing, or be prepared by inserting it into a multi-dose container. The term, "pharmaceutically effective dose" refers to an amount which can show a desired effect of the active ingredient (that is, the anti-MUC1 antibody or antigen binding fragment, or the anti-MUC1/anti-CD3 bispecific antibody or antigen binding fragment thereof), in other words, an effect of preventing and/or treating cancer, and it may be variously prescribed by factors such as the preparation method, administration method, patient's age, body weight, gender, morbid condition, food, administration time, administration route, excretion rate, and reaction reactivity.

The pharmaceutical composition may be prepared in a single dose form or be prepared by inserting it into a multidose container, by formulating it using a pharmaceutically acceptable carrier and/or excipient. At this time, the formulation may be in a solution in oil or aqueous media, suspension, syrup or emulsion form, or may be in an extract, powder, powdered agent, granule, tablet or capsule form, and may comprise a dispersant or stabilizer additionally.

Furthermore, the pharmaceutical composition may be administered as an individual therapeutic agent or administered in combination with another therapeutic agent, and it may be administered with a conventional therapeutic agent sequentially or simultaneously.

On the other hand, the pharmaceutical composition includes an antibody or antigen binding fragment thereof, so it may be formulated as an immunoliposome. Liposome comprising an antibody may be prepared according to a method widely known in the art. The immunoliposome is a lipid composition comprising phosphatidylcholine, cholesterol, and polyethylene glycol-derivatized phosphatidyl ethanolamine, and may be prepared by reverse-phase evaporation method. For example, a Fab' fragment of an antibody may be conjugated to liposome through a disulfide-replacement reaction. A chemotherapeutic agent such as doxorubicin may be further comprised in the liposome.

The administration subject of the pharmaceutical composition or the patient subjected to administration of the method for prevention and/or treatment may be a mammal, for example, a primate such as a human, a monkey, and the like, or a rodent such as a rat, a mouse, and the like, but not limited thereto, and may be a cancer patient with resistance to an antagonist against a conventional anticancer agent, for example, the target cell membrane protein (for example, MUC1).

### Method for preparation of polypeptide, recombinant vector, and antibody

Other embodiment of the present invention provides a polynucleotide encoding the polypeptide described above. In one specific embodiment, the polynucleotide may encode a polypeptide comprising at least one amino acid sequence selected from the group consisting of SEQ ID NOs: 2 to 6, SEQ ID NOs: 8 to 15, SEQ ID NOs: 17 to 18, SEQ ID NOs: 20 to 26, SEQ ID NOs: 28 to 32, and SEQ ID NOs: 34 and SEQ ID NOs: 38.

In other specific embodiment, the polynucleotide may encode a polypeptide comprising an amino acid sequence selected from the group consisting of SEQ ID NOs: 77 to 96.

Other embodiment provides a recombinant vector comprising the polynucleotide.

Other embodiment provides a recombinant cell transformed with the recombinant vector.

The term, "vector" refers to a means to express a target gene in a host cell. For example, it includes virus vectors such as a plasmid vector, a cosmid vector and a bacteriophage vector, an adenovirus vector, a retrovirus vector and an adeno-associated virus vector. The vector which can be used as the recombinant vector may be constructed by engineering a plasmid (for example, pSC101, pGV1106, pACYC177, ColE1, pKT230, pME290, pBR322, pUC8/9, pUC6, pBD9, pHC79, pIJ61, pLAFR1, pHV14, pGEX series, pET series and pUC19, etc.), phage (for example, λgt4λB, λ-Charon, λΔz1 and M13, etc.) or virus (for example, SV40, etc.) often used in the art, but not limited thereto.

In the recombinant vector, the polynucleotide may be operatively linked to a promoter. The term, "operatively linked" refers to functional binding between a nucleotide expression regulatory sequence (for example, promoter sequence) and another nucleotide sequence. The regulatory sequence may regulate transcription and/or translation of another nucleotide sequence by being "operatively linked".

The recombinant vector may be typically constructed as a vector for cloning or a vector for expression. The vector for expression may use common one used for expressing a foreign protein in a plant, animal or microorganism. The recombinant vector may be constructed by various methods known in the art.

The recombinant vector may be constructed using a prokaryotic cell or eukaryotic cell as a host. For example, when the used vector is an expression vector, and a prokaryotic cell is used as a host, it is common to comprise a strong promoter which can progress transcription (for example, pLλ promoter, CMV promoter, trp promoter, lac promoter, tac promoter, T7 promoter, etc.), a ribosome binding site for initiation of translation and a transcription/translation termination sequence. When a eukaryotic cell is used as a host, the replication origin acting in the eukaryotic cell comprised in the vector includes f1 replication origin, SV40 replication origin, pMB1 replication origin, adeno-replication origin, AAV replication origin and BBV replication origin and the like, but not limited thereto. In addition, a promoter derived from genome of a mammal cell (for example, metallothionein promoter) or a promoter derived from a mammal virus (for example, adenoviral late promoter, vaccinia virus 7.5K promoter, SV40 promoter, cytomegalovirus promoter and tk promoter of HSV) may be used, as the transcription termination sequence, it generally has a polyadenylated sequence.

The recombinant cell may be obtained by introducing the recombinant vector into an appropriate host cell. As the host cell, any host cell known in the art as a cell capable of cloning or expressing the recombinant vector stably and continuously may be used, and the prokaryotic cell includes for example, E. coli JM109, E. coli BL21, E. coli RR1, E. coli LE392, E. coli B, E. coli X 1776, E. coli W3110, Bacillus sp. Strains such as Bacillus subtilis and Bacillus thuringiensis, and Enterobacteriaceae strains such as Salmonella typhimurium, Serratia marcescens, and various Pseudomonas sp. And the like, and when it is transformed into a eukaryotic cell, as the host cell, yeast (Saccharomyces cerevisiae), insect cells, plant cells and animal cells, for example, Sp2/0, CHO (Chinese hamster ovary) K1, CHO DG44, PER.C6, W138, BHK, COS-7, 293, HepG2, Huh7, 3T3, RIN, MDCK cell lines and the like may be used, but not limited thereto.

The delivery (introduction) of the polynucleotide or recombinant vector comprising the same into a host cell, may use a delivery method widely known in the art. As the delivery method, for example, when the host cell is a prokaryotic cell, CaCl2 method or electroporation method or the like may be used, and when the host cell is a eukaryotic cell, microinjection, calcium phosphate precipitation, electroporation, liposome-mediated transfection and gene bombardment and the like may be used, but not limited thereto.

The method for selecting the transformed host cell may be easily conducted according to a method widely known in the art, using a phenotype expressed by a selectable marker. For example, when the selectable marker is a specific antibiotic resistant gene, by culturing a transformant in a medium containing the antibiotic, the transformant may be easily selected.

Other embodiment provides a method for preparation of the polypeptide or anti-MUC1 antibody or antigen binding fragment, or the anti-MUC1/anti-CD3 bispecific antibody or antigen binding fragment thereof comprising expressing the polynucleotide in the recombinant cell. The expressing the polynucleotide may comprise culturing the recombinant cell under conditions of allowing expression of polynucleotide comprised herein. The method for preparation of the polypeptide or anti-MUC1 antibody or antigen binding fragment, or the anti-MUC1/anti-CD3 bispecific antibody or antigen binding fragment thereof may further comprise isolating and/or purifying the polypeptide or anti-MUC1 antibody or antigen binding fragment, or the anti-MUC1/anti-CD3 bispecific antibody or antigen binding fragment thereof, after the expressing or culturing.

### [ADVANTAGEOUS EFFECTS]

The anti-MUC1 antibody, and anti-MUC1/anti-CD3 bispecific antibody can effectively inhibit growth of cancer cells, and therefore, they have an excellent effect for prevention or treatment of cancer.

### [BRIEF DESCRIPTION OF THE DRAWINGS]

FIG. 1 shows the binding reaction of the pancreatic cancer-specific MP1 monoclonal antibody to peripheral blood and normal pancreatic epithelial cells and pancreatic cancer cells. (granulocytes, lymphocytes, monocytes, normal pancreatic epithelial cells (H6C7), 5 kinds of pancreatic cancer cell lines (AsPC1, BxPC3, Capan-1 Capan-2, PANC-1))
FIG. 2A is the result of immunohistochemistry (IHC) in pancreatic cancer tissue, and FIG. 2B shows the IHC result in breast cancer cells, and T in the drawing shows tumor sites.
FIG. 3 shows the western blot result of the MP1 monoclonal antibody.
FIG. 4 shows the amino acid sequence of MUC1 analyzed by LC-MS/MS and identified.
FIG. 5 shows the reactivity assay (ELISA) of the MP1 monoclonal antibody against the MUC1 antigen.
FIG. 6 shows the reactivity assay (IP-Western blot (IB)) of the MP1 monoclonal antibody against the MUC1 antigen.
FIG. 7A and FIG. 7B show the antigen quantification result on the surface of the pancreatic cancer cell lines responding to MP1.
FIG. 7C and FIG. 7D show the epitope mapping result of the MP1 monoclonal antibody using the circular peptide array method.
FIG. 8 shows the binding reaction of the MP1 humanized antibody to pancreatic cancer cells, and the staining cell line is Capan-2, and the heavy chain and light chain antibody gene combination and cell binding positivity (%) are described.
FIG. 9 shows the structure of the LCC type anti-MUC1/OKT3 antibody.
FIG. 10A and FIG. 10B are results showing cell surface binding of the LCC type anti-MUC1/OKT3 antibody to AsPC1, a pancreatic cancer cell.
FIG. 11 is the result showing cytotoxicity of the LCC type anti-MUC1/OKT3 antibody against Capan-2 and AsPC1, pancreatic cancer cells.
FIG. 12 shows the structure of the LCN type anti-MUC1/OKT3 antibody.
FIG. 13 is the result showing cytotoxicity of the LCN type anti-MUC1/OKT3 antibody against Capan-1, a pancreatic cancer cell.
FIG. 14 is the result showing cytotoxicity of the LCN type anti-MUC1/OKT3 antibody against AsPC1, a pancreatic cancer cell.
FIG. 15 is the result showing cytotoxicity of the LCN type anti-MUC1/OKT3 antibody against Capan-2 and AsPC1, pancreatic cancer cells, SNU-601, a gastric cancer cell, and T47D, a breast cancer cell.
FIG. 16 is the result confirming the therapeutic effect of cancer cells by administering the anti-MUC1/OKT3 antibody in the NOG mouse animal model in which tumor is induced by injecting AsPC-1, a pancreatic cancer cell.

### [MODE FOR INVENTION]

Hereinafter, the present invention will be described in more detail by the following examples. However, they are intended to illustrate the present invention only, but the scope of the present invention is not limited by these examples.

### Example 1. MP1 monoclonal antibody discovery

In order to develop a new antibody specific to pancreatic cancer, the following experiment was performed, and the developed antibody was partially expressed not only in pancreatic cancer but also breast cancer and gastric cancer, and was named MP1 as a solid cancer-specific antibody.

### Example 1-1. Method for design and selection of target site for manufacturing MP monoclonal antibody

In order to develop monoclonal antibodies against specific antigens expressed in pancreatic cancer, various pancreatic cancer cell lines, AsPC1, BxPC3, Capan-1 Capan-2, PANC-1 were immunized into a mouse, and then monoclonal antibodies against them were established by a cell fusion method. After that, during the hybridoma selection process, antibodies that were positive for the pancreatic cancer cell lines and negative for the normal pancreatic cell line H6C7 and peripheral blood were selected.

### Example 1-2. Hybridoma preparing antibody selection

In order to develop monoclonal antibodies specific to pancreatic cancer, 5 kinds of pancreatic cancer cell lines (AsPC1, BxPC3, Capan-1 Capan-2, PANC-1) were mixed in equal numbers, and were immunized by injecting them into an intraperitoneal cavity (IP) as much as 1×10⁷ per one 6-week-old Balb/c female mouse at intervals of 3 weeks three times. The spleen of the immunized mouse as described above was excised and a single cell suspension was obtained and washed with RPM11640 twice, and then, 0.4% trypan blue (sigma) was mixed at 1:1(v/v) and then the number of cells was counted using a trypan blue staining method that measures unstained cells with a microscope. X63 mouse myeloma cell line (ATCC CRL-1580) was used as a cell fusion partner cell, and it was washed in the same manner with the spleen cells, and then the number of cells was counted. After mixing the myeloma cells and spleen cells, PEG (polyethylene glycol) 1500 was added to fuse the cells, and then the cells were centrifuged. The fused hybridoma cells were suspended in RPMI (20% FBS, hypoxanthine-aminopterin-thymidine) containing 1xHAT and 150 ul was aliquoted to a 96-well plate, and then they were cultured in a 37 °C 5% CO2 incubator. After the fusion, a selection process was conducted in a medium comprising HAT for a certain period of time, and when a well in which a colony was formed was observed, it was replaced with an HT medium, and cultured in a 37 °C 5% CO2 incubator for 48 hours, and then for the normal pancreatic cell line H6C7 and 5 kinds of pancreatic cancer cell lines, AsPC1, BxPC3, Capan-1 Capan-2, PANC-1 used for immunization, fluorescent staining was performed, respectively, as follows, and they were analyzed by flow cytometry. The hybridoma culture supernatant was added to the prepared cells by 100 ul each and reacted at 4 °C for 30 minutes, and then PBS 3ml was added and unbound antibodies were washed by centrifuging at 1700 rpm for 3 minutes and to confirm the bound antibodies, a secondary antibody, goat anti-Mouse Ig-FITC (Jackson ImmunoResearch, USA) was diluted by 200 times and added, and then reacted at 4 °C for 15 minutes, and then washed with PBS 3 ml by the same method as above, and then measured using flow cytometry.

### Example 1-3.

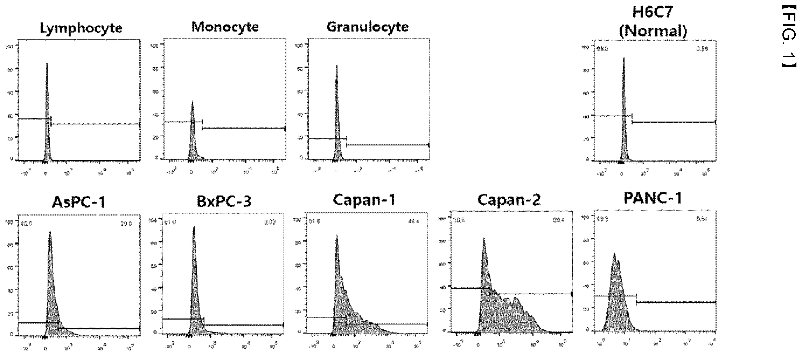

In order to confirm the degree of binding to peripheral blood, the hybridoma supernatant was added to PBMC (peripheral blood mononuclear cells, Zenbio) by 100 ul and it was reacted at 4 °C for 30 minutes, and then PBS 3 ml was added and unbound antibodies were washed by centrifuging at 1700 rpm for 3 minutes, and to confirm the bound antibodies, a secondary antibody, goat anti-Mouse Ig-FITC (Jackson ImmunoResearch, USA) was diluted by 200 times and added, and then reacted at 4 °C for 15 minutes, and then washed with PBS 3ml by the same method as above, and then measured by flow cytometry, and the result was described (FIG. 1). FIG. 1 shows the result of the reactivity of the MP1 monoclonal antibody specific to pancreatic cancer with peripheral blood and the normal pancreatic epithelial cell (H6C7) and various kinds of pancreatic cancer cells.

As shown in FIG. 1, the monoclonal antibody (hereinafter, named MP1) which was positive for various pancreatic cancer cell lines and was negative for all the normal pancreatic epithelial cell, H6C7 and granulocytes, lymphocytes and monocytes of the peripheral blood, was selected, and finally, through limiting dilution, the MP1 hybridoma cell of the single colony was obtained.

### Example 2. Antibody expression analysis in various cell lines

### Example 2-1. Antibody expression in various cell lines

Binding of the MP1 monoclonal antibody in various cancer cell lines obtained through KCLB (Korean Cell Line Bank) and ATCC (American Type Culture Collection) was confirmed by flow cytometry. Specifically, the cancer cell lines were cultured using RPMI1640 medium or DMEM medium in which 10%heat inactivated FBS was added using a 5% CO2 incubator at 37 °C according to the culture medium and method suggested from KCLB (Korean Cell Line Bank) and ATCC (American Type Culture Collection). In addition, the human pancreatic epithelial cell line, H6C7 was purchased from kerafast company, and was cultured using Keratinocyte SFM (Invitrogen Cat#:17005042) using a 5% CO2 incubator at 37 °C.

The MP1 monoclonal antibody of the present invention was added to the cultured various cancer cells, respectively, and they were reacted at 4 °C for 30 minutes and then washed with PBS, and FITC-conjugated goat anti mouse IgG (Jackson ImmunoResearch, USA) was added and they were cultured at 4 °C for 15 minutes. They were washed with PBS again, and then were analyzed with FACS caliber (Becton Dickinson, USA) to confirm the reactivity of the MP1 antibody in various solid cancer cell lines (Table 9).

As shown in Table 9 and Table 10 below, it was confirmed that the MP1 monoclonal antibody highly bound to the pancreatic cancer and some of gastric cancer, breast cancer, lung cancer cell lines, and skin cancer and prostate cancer cell lines, while it did not bind or weakly bind in T cells and B cells comprising the normal pancreatic epithelial cell line, H6C7, and brain cancer, biliary tract cancer, and colorectal cancer cell lines. PBMC represents that it is negative in normal blood cells.

MP1 showed a positive response in various pancreatic cancer cells, and showed a negative response in the normal pancreatic epithelial cell, and therefore, it shows that it can be used as a pancreatic cancer therapeutic agent.

**[Table 9]**

| Origin | Cell line | MP1 | Origin | Cell line | MP1 |
|---|---|---|---|---|---|
| PBMC | lymphocyte | - | Cervix | HeLa S3 | ++ |
| | monocyte | - | | SiHa | + |
| | granulocyte | - | Skin | A431 | +++ |
| T cell | H9 | - | Melanoma | HS294T | - |
| | CEM 7 | - | | MDAMB435 | - |
| | CEM20 | - | | Sk-MEL-28 | + |
| | Molt4 | - | Gland & Breast | CAMA-1 | +++ |
| | Jurkat | - | | MCF7 | ++ |
| B cell | ARH-77 | - | | ZR-75-1 | + |
| | Daudi | - | | T-47D | +++ |
| | Ramos | - | | JIMT-1 | - |
| | RPMI 8226 | - | | MDAMB453 | - |
| | Raji | - | | SK-BR3 | - |
| | BJAB | + | | BT20 | ++ |
| Myeloid | U937 | - | | MDAMB231 | - |
| | THP-1 | + | | MDAMB468 | - |
| | MOLM-13 | - | | HS578T | - |
| | kasumi-1 | - | Colon | HT-29 | - |
| | HL-60 | - | | COLO 205 | - |
| Erythroid | HEL | - | | LS 174T | - |
| Bone marrow | K562 | - | | SNU C1 | + |
| | KG-1 | - | | SW620 | - |
| Thyroid Brain | TPC-1 | +++ | | DLD-1 | - |
| | Daoy | - | | SW-480 | - |
| | H4 | - | | HCT 116 | - |
| | Hs683 | - | | SNU C2A | - |
| | SNB-19 | - | | SNUC4 | - |
| Lung | NCI H417 | - | Stomach | SNU 719 | - |
| | HCC-1195 | + | | KATOIII | + |
| | HCC-1588 | - | | SNU668 | - |
| | HCC-2279 | - | | SNU16 | - |
| | HCC-95 | - | | Hs 746T | - |
| | NCI-H23 | - | | AGS | +++ |
| | calu-3 | - | | MKN 28 | - |
| | A549TA | + | | MKN 74 | - |
| | NCI-H460 | - | | MKN-45 | - |
| | NCI-H1299 | + | | SNU601 | +++ |
| | SK-MES-1 | - | Prostate | PC-3 | - |
| | L132 | +++ | | DU145 | +++ |
| Liver | HEPG2 | - | Pancreas | H6C7 | - |
| | Hep3B.1-7 | - | | AsPC-1 | +++ |
| | SK-HEP-1 | - | | BxPC-3 | - |
| | PLC/PRF/5 | - | | Capan-1 | + |
| | Huh-7 | - | | Capan-2 | +++ |
| biliary tract | SNU-308 | - | | MIA PaCa-2 | - |
| | SNU-245 | - | | PANC-1 | - |
| Kidney | 786-O | - | | | |
| The percentage of MP1 positive cells were calculated by FACS analysis. | | | | | |
| -: less than 20% of positive cells, +20-30%, ++: 40-70%, +++: 60-100% | | | | | |

**[Table 10] Types of solid cancers that are positive for MP1 antibody**

| Solid cancer types | Number of positive cancer cell lines / Number of stained cancer cell lines |
|---|---|
| Pancreas | 3/6 |
| Breast | 5/11 |
| Stomach | 2/10 |
| Cervix | 2/2 |
| Prostate | 1/2 |
| Lung | 4/11 |
| Thyroid | 1/1 |
| Skin | 1/1 |
| Melanoma | 1/3 |

### Example 2-2. IHC (Immunohistochemistry) assay

Distribution of binding antigens of the MP1 monoclonal antibody in human pancreatic cancer and breast cancer tissues was confirmed by the immunohistochemistry (IHC) method. The human pancreatic cancer and breast cancer tissues were obtained from Chungbuk National University Hospital, and paraffin sections were manufactured at the Department of Pathology at Chungbuk National University Hospital.

The human breast cancer tissue to be stained was fixed in formalin using a common method and then embedded in paraffin to manufacture microarray sections. Then, it was placed on a slide to produce a single tissue slide for staining reading. Subsequently, using the MP1 antibody, immunohistochemical staining was performed by the following method. A deparaffinization process was performed by treating xylene three times for 10 minutes each, and treating ethanol in the order of 100%, 80%, and 50%, and finally, washing with distilled water. After washing with 1 X TBS, the slide was placed in 10 mM Citric acid (pH 6.0) antigen regeneration solution and antigen regeneration was performed in a microwave for 20 minutes. The temperature of the slid was slowly lowered using tap water, and it was stored in 1 X TBS for 10 minutes. As the primary antibody, the mouse MP1 antibody was treated to the tissue and it was reacted at a room temperature for 1 hour and then washed with 1 X TBS three times and as the secondary antibody, the anti-mouse antibody-HRP conjugate was reacted at a room temperature for 30 minutes. It was washed with 1 X TBS three times and DAB color development was performed for 1 minute and 30 seconds, and washed with 1 X TBS three times, and counter staining was conducted for 45 seconds and the slide was put in flowing tap water and washed for 10 minutes. The dehydration process was carried out in the reverse order of the deparaffinization process, and it was sealed and microscopic reading or photographing was conducted.

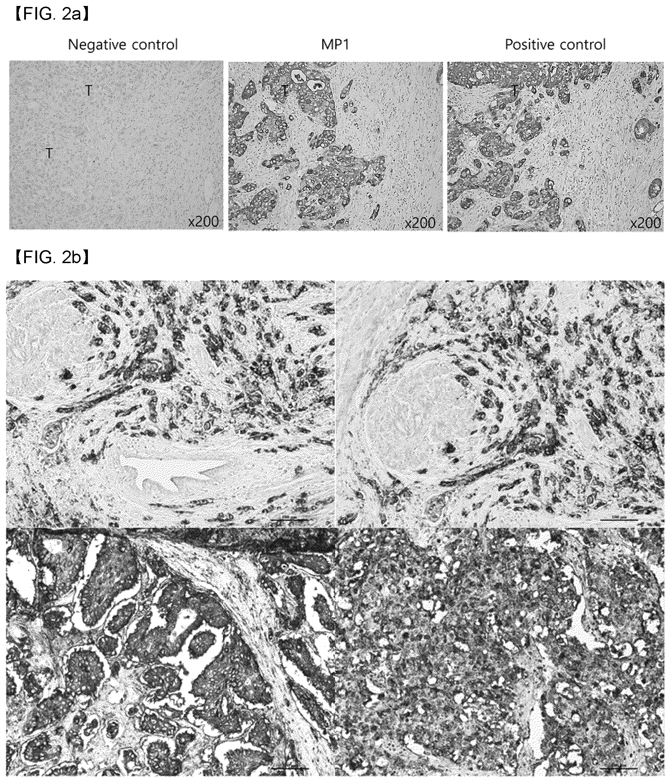

As shown in FIG. 2A and FIG. 2B, it was confirmed that the antigen recognized by the MP1 monoclonal antibody was not distributed in the normal tissue, and it was expressed specifically to the cancer tissue.

### Example 3. Antigen analysis against MP1 monoclonal antibody

### Example 3-1. Confirmation of antigen size of MP1 monoclonal antibody

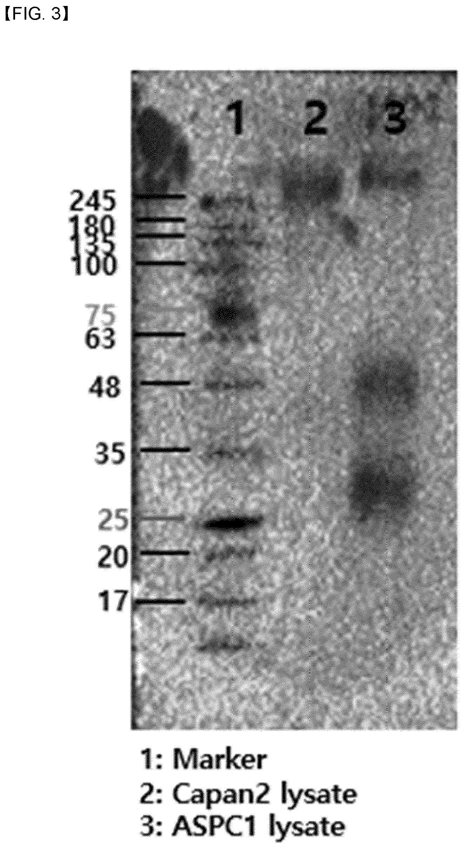

In order to investigate the protein size of the antigen recognized by the MP1 monoclonal antibody of the present invention, western blot was performed. The pancreatic cancer cell line with high antigen expression against MP1 was lysed in a cell lysis solution comprising protease inhibitor cocktail (Sigma, Cat. #: 1136170001) (NP40 cell lysis buffer, Thermo Fisher Scientific), and then electrophoresis was performed in SDS polyacrylamide gel, to transfer to Immobilon-P membrane (Millipore Corporation, Billerica, MA, USA). After that, the purified MP1 monoclonal antibody was treated to membranes, and then it was color-developed in ECL system (GE Healthcare, Buckinghamshire, UK) to confirm bands (FIG. 3). As could be confirmed in FIG. 3, as the result of the measurement, the size of the antigen recognized by the MP1 monoclonal antibody was near approximately 300 kDa.

### Example 3-2. Separation analysis of MP1 monoclonal antigen

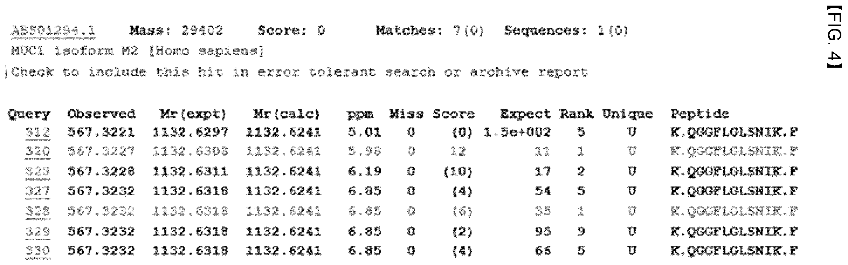

In order to identify the antigen recognized by the MP1 monoclonal antibody of the present invention, immunoprecipitation was performed. First, Capan-2 cell line, a cell line highly reactive to the MP1 monoclonal antibody, was lysed at 1×10⁷ cells/ml in a cell lysis solution (NP40 cell lysis buffer, Thermo Fisher Scientific), and then cell residues were removed through centrifugation and the supernatant was collected and used as a material for separation of the antigen recognized by the MP1 antibody. The MP1 monoclonal antibody was added to Protein-G Sepharose, and mixed at 4 °C for 4 hours, and then washed with 1X PBS twice, and then the previously prepared Capan-2 lysate was added and mixed at 4 °C for 4 hours, and then washed with 1X PBS twice. For the immune complex obtained therefrom, SDS-polyacrylamide gel electrophoresis was performed, and then the gel was stained with Coomassie Brilliant Blue G-250, and then the band presumed as the antigen against MP1 around 300 kDa was cut and LC-MS/MS analysis was requested for identification to ProteomTech. For the identification, the result obtained by analyzing with LC-MS/MS was processed with MASCOT. As a result of selecting by limiting to the antigen with a protein size around 300kDa while expressing 100 kinds of protein hits obtained by a series of analyses as above on the cell surface, MUC1 could be selected as the antigen against the MP1 monoclonal antibody (FIG. 4). The peptide sequence isolated from MP1 is QGGFLGLSNIK (SEQ ID NO: 107).

### Example 3-3. Verification of antigen identification result of MP1 monoclonal antibody by ELISA

In order to verify the antigen identification result obtained from LC-MS/MS, the reactivity of the MUC1 monoclonal antibody against the recombinant protein MUC1 (Sino Biological, Cat. #: 12123-HCCH) was confirmed by ELISA and Western blotting assay.

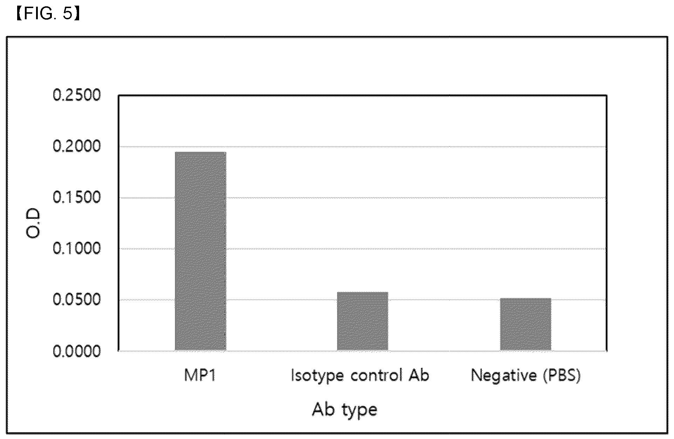

100 ng of the recombinant protein MUC1 per well was added to Maxisorp ELISA plate and it was reacted at 37 °C for 1 hour to coat the antigen, and then 200 ul of 1x blocking solution (sigma) per well was added and it was reacted at 37 °C for 1 hour for blocking. The MP1 monoclonal antibody and control antibody, PBS 100 ul were added to the prepared plate, respectively, and then they were reacted at 37 °C for 1 hour and washed with PBS and unbound antibodies were removed. After that, Goat anti-Mouse IgG-HRP (Jackson ImmunoResearch, USA) was diluted and added, and reacted for 30 minutes, and then washed with PBS, and then 50 ul of TMB solution per well was added and reacted for 10 minutes, and then 50 ul of sulfuric acid was added and the reaction was stopped and the absorbance was measured at 450 nm. The MP1 monoclonal antibody against the recombinant protein MUC1 showed the reactivity as FIG. 5. FIG. 5 is the result of verifying that the antigen against the MP1 monoclonal antibody is MUC1 by ELISA.

### Example 3-4. Verification of antigen identification result of MP1 monoclonal antibody through IP and IB

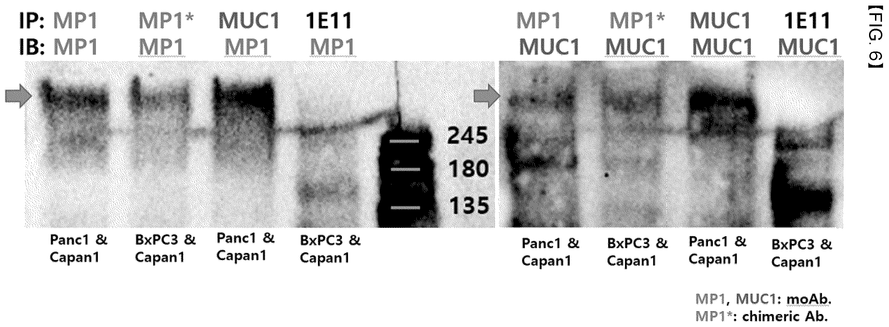

In the above experiment, that the MP1 monoclonal antibody recognized MUC1 as the antigen was verified by western blot once more. As same as described in Example 3-2, various pancreatic cancer cell lines, BXPC3, Capan-1, Capan-2, PANC1, lysis solutions were prepared, and the commercialized MUC1 antibody (BioXCell, Cat. # C595 (NCRC48)) and MP1 monoclonal antibody were mixed to Protein-G Sepharose at 4 °C for 4 hours, respectively, and then washed with 1X PBS twice, and then the previously prepared lysate was added and mixed at 4 °C for 4 hours, and then washed with 1X PBS twice. The immune complex obtained therefrom was boiled for 10 minutes, and then it was under electrophoresis in 8 % SDS polyacrylamide gel, and transferred to Immobilon P membrane (Millipore Corporation, Billerica, MA, USA). After that, the purified MP1 monoclonal antibody was treated to membranes, and then it was color-developed in ECL system (GE Healthcare, Buckinghamshire, UK) to confirm bands. As a result of western blotting with the MP1 antibody for the immune complex obtained by reacting the commercialized MUC1 antibody and pancreatic cancer cell lines, a band could be confirmed at the 300 kDa position as same as Capan-2 lysate alone and the immune complex sample of MP1 and pancreatic cancer cell line lysate. FIG. 6 is the result of verifying that the antigen against the MP1 monoclonal antibody is MUC1 by western blot.

### Example 4. Quantification of cell surface antigens

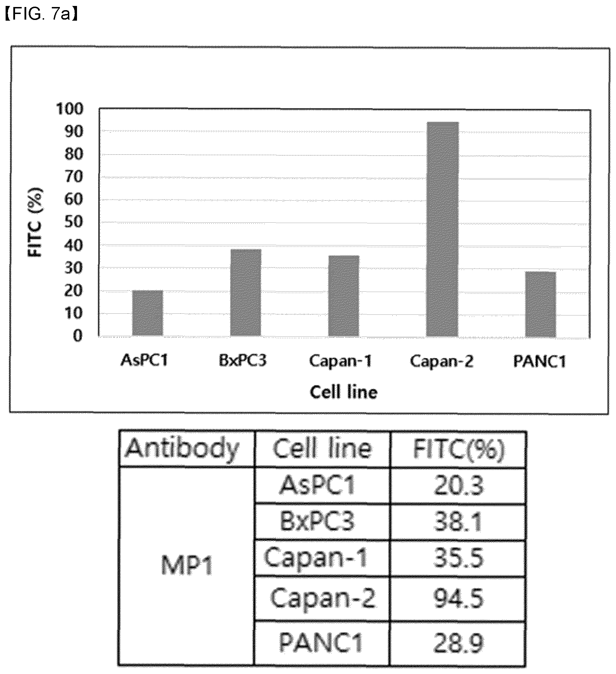

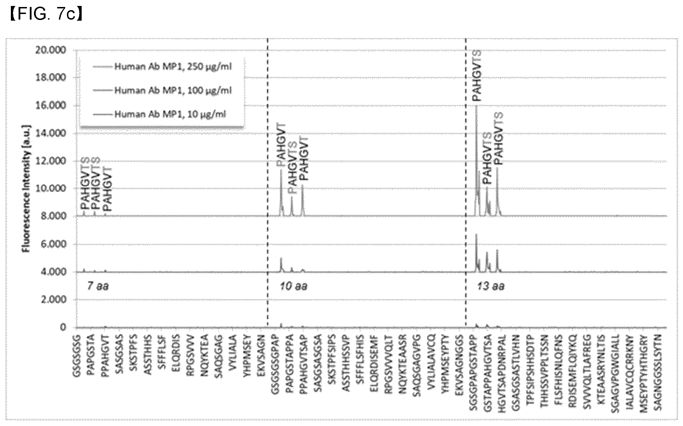

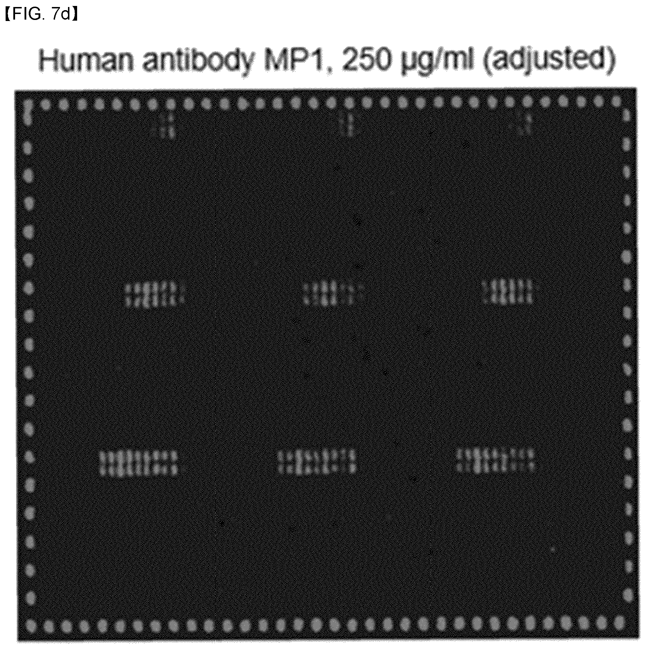

The expression level of antigens binding to the MP1 monoclonal antibody on the pancreatic cancer cell surface was measured using a quantitative flow cytometry kit for measuring cell surface antigens, QIFIKIT (Dako, Cat.#: K0078). The antibody binding affinity (ABC) of the cell lines could be calculated by comparison of mean fluorescence intensity (MFI) of beads through a calculation graph. FIG. 7A is the result showing the expression level of antigens reacting to MP1 detected on the cell surface of various pancreatic cancer cell lines (AsPC1, BxPC3, Capan-1, Capan-2, PANC1) using QIFIKIT for the MP1 monoclonal antibody.

### Example 5. Epitope mapping

The following is a partial sequence of the MUC-1 antigen (amino acids 895 - 1264^{th} amino acid sequence) used for epitope mapping (Table 11). The epitope mapping was performed through circular peptide array by selecting the region comprising the amino acid sequence of the recombinant MUC-1 antigen used for antigen identification verification.

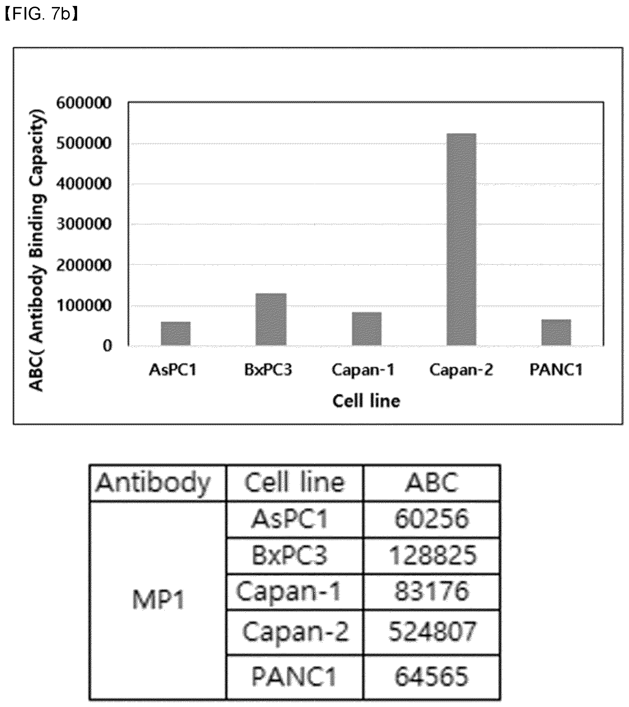

As could be confirmed in FIG. 7B, the MP1 antibody bound to a peptide comprising the PAHGVTS sequence in a dose-dependent manner, and thus, the antigen binding site of MP1, that is, an epitope was identified as PAHGVTS (SEQ ID NO: 109).

**[Table 11]**

| |
|---|
| MUC-1 Isoform 2 (identifier: P15941-2) (895th amino acid to 1264th amino acid) |
| PAPGSTAPPAHGVTSAPDTRPAPGSTAPPAHGVTSAPDTRPAPGSTAPPAHGV TSAPDNRPALGSTAPPVHNVTSASGSASGSASTLVHNGTSARATTTPASKSTP FSIPSHHSDTPTTLASHSTKTDASSTHHSSVPPLTSSNHSTSPQLSTGVSFFFL |
| SFHISNLQFNSSLEDPSTDYYQELQRDISEMFLQIYKQGGFLGLSNIKFRPGSV VVQLTLAFREGTINVHDVETQFNQYKTEAASRYNLTISDVSVSDVPFPFSAQSG AGVPGWGIALLVLVCVLVALAIVYLIALAVCQCRRKNYGQLDIFPARDTYHPMSE YPTYHTHGRYVPPSSTDRSPYEKVSAGNGGSSLSYTNPAVAATSANL (SEQ ID NO: 108) |

### Example 6. Analysis of sequence and isotype of MP1 antibody variable regions

### Example 6-1. Isotype determination

In order to determine the isotype of the MP1 monoclonal antibody prepared in Example 1, it was analyzed with mouse immunoglobulin isotyping ELISA kit (BD Biosciences, USA). As a result, the MP1 monoclonal antibody was identified as mouse IgG2a/kappa light chain.

### Example 6-2. MP1 antibody variable region sequence analysis

The MP1 antibody gene was cloned using Mouse Ig-Primer Set (Merck Millipore, Cat. #:69831). PCR was performed using Mouse Ig-Primer Set from RNA isolated from the MP1 hybridoma, and this was inserted into the pGEM-T vector (Promega, Cat. #: A3600), and then the DNA base sequence was confirmed through sequencing, and the mouse antibody gene was confirmed through IMGT site (www.imgt.org). Table 12 shows the analyzed sequences in the heavy chain and light chain variable regions of the MP1 antibody. The thickly underlined portions in the amino acid sequences described below represent complementarity determining regions. In the following Table 12, the sequence positions are written on the basis of Kabat numbering system.

**[Table 12]**

| Name | Sequence | Sequenc e position | Len gth | SEQ ID NO: |
|---|---|---|---|---|
| MP1 VH-FR1 | EVKVDETGGGLVQPGRPMKLSCVVSGI TFS | 1-30 | 30 | 39 |
| MP1 VH-CDR1 | DYWMN | 31-35 | 5 | 2 |
| MP1 VH-FR2 | WVRQSPEKGLEWVA | 36-49 | 14 | 49 |
| MP1 VH-CDR2 | QIRNKPYNYETYYSNSVKG | 50-68 | 19 | 8 |
| MP1 VH-FR3 | RFTISRDDSKSSVYLQMNNLRAEDMGI YYCTW | 69-100 | 32 | 52 |
| MP1 VH-CDR3 | AMITSDGYAMDY | 101-112 | 12 | 17 |
| MP1 VH-FR4 | WGQGTSVTVSS | 113-123 | 11 | 59 |
| MP1 Ab VH | EVKVDETGGGLVQPGRPMKLSCVVSGI | 1-123 | 123 | 77 |
| | TFSDYWMNWVRQSPEKGLEWVAQIRN KPYNYETYYSNSVKGRFTISRDDSKSS VYLQMNNLRAEDMGIYYCTWAMITSD GYAMDYWGQGTSVTVSS | | | |
| MP1 VL-FR1 | DIVMSQSPSSLAVSVGEKVTLIC | 1-23 | 23 | 61 |
| MP1 VL-CDR1 | KSSQSLLLSSNQKNYLA | 24-40 | 17 | 20 |
| MP1 VL-FR2 | WYQQKPGQSPKLLIY | 41-55 | 15 | 66 |
| MP1 VL-CDR2 | WASTRES | 56-62 | 7 | 28 |
| MP1 VL-FR3 | GVPDRFTGSGSGTDFTLTINSVKAEDL AVYYC | 63-94 | 32 | 70 |
| MP1 VL-CDR3 | QQCFSYPLT | 95-103 | 9 | 34 |
| MP1 VL-FR4 | FGAGTKLELK | 104-113 | 10 | 76 |
| MP1 Ab VL | DIVMSQSPSSLAVSVGEKVTLICKSSQS LLLSSNQKNYLAWYQQKPGQSPKLLIY WASTRESGVPDRFTGSGSGTDFTLTIN SVKAEDLAVYYCQQCFSYPLTFGAGTK LELK | 1-113 | 113 | 87 |

### Example 7. Development of MP1 humanized antibodies

Humanized antibodies in which MP1 variable regions and other parts were replaced with human Fc were manufactured to reduce immunogenicity shown by a human anti-mouse antibody (HAMA) reaction and secure an antibody with high druggability, before preparing a bispecific antibody, when a mouse-originated antibody was administered into a human body. It was confirmed that the selected humanized antibody had similar antigen specificity and affinity to the original mouse-derived MP1 antibody.

### Example 7.1 Selection of recombinant antibody sequence by in silico humanization

CDR region sequences of each of the heavy chain and light chain of the mouse antibody MP1 (heavy chain amino acid sequence: SEQ ID NO 77; light chain amino acid sequence: SEQ ID NO: 87) are VH-CDR1: SEQ ID NO: 2 (DYWMN); VH-CDR2: SEQ ID NO: 8 (QIRNKPYNYETYYSNSVKG); VH-CDR3: SEQ ID NO: 17 (AMITSDGYAMDY); VL-CDR1: SEQ ID NO: 20 (KSSQSLLLSSNQKNYLA); VL-CDR2: SEQ ID NO: 28 (WASTRES); VL-CDR3: SEQ ID NO: 34 (QQCFSYPLT). Humanized antibodies with equal or excellent antigen binding affinity while maintaining the mouse antibody MP1 heavy chain and light chain amino acid sequences similarly as possible were selected by recombining sequence regions for the framework region based on the sequence of the germline encoding the human antibody gene by in silico method. The human antibody germline gene used as the backbone of the humanized recombinant antibody sequence due to the highest similarity to each sequence of the heavy chain and light chain of the mouse antibody MP1 is shown as the following Table 13.

The amino acid sequences of the amino acid sequences of the amino acid sequences of the heavy chain variable regions and the amino acid sequences of the light chain variable regions of the selected humanized antibody were shown in the following Table 14. The thickly underlined portions in the amino acid sequences described below represent complementarity determining regions.

**[Table 13]**

| Human Ab Germline | |
|---|---|
| Heavy chain | Light chain |
| IGHV3-49*04 | IGKV4-1*01 |
| IGHV3-7*01 | IGKV2-28*01 |
| IGHV3-33*01 | IGKV3-20*01 |

**[Table 14]**

| Heavy chain variable region | Amino acid sequence | SEQ ID NO: |
|---|---|---|
| VH1 (MP1-H349-V1) | EVKVVESGGGLVQPGRSLRLSCTASGITFSDYWM NWVRQAPGKGLEWVGQIRNKPYNYETYYSQSVK GRFTISRDDSKSIAYLQMSSLKTEDTAVYYCTWAMI TSDGYAMDYWGQGTLVTVSS | 78 |
| VH2 (MP1-H349-V2) | EVQLVESGGGLVQPGRSLRLSCTASGITFGDYWM NWVRQAPGKGLEWVGQIRSKPYNYETYYAASVKG RFTISRDDSKSIAYLQMSSLKTEDTAVYYCTWAMIT SDGYAMDYWGQGTLVTVSS | 79 |
| VH3 (MP1-H349-V3) | EVQLVESGGGLVQPGRSLRLSCTASGFTFGDYWM SWVRQAPGKGLEWVGFIRSKAYGGTTEYAASVKG RFTISRDDSKSIAYLQMSSLKTEDTAVYYCTWAMIT SDGYAFDYWGQGTLVTVSS | 80 |
| VH4 (MP1-H37-V1) | EVKVVESGGGLVQPGGSLRLSCAASGITFSDYWM NWVRQAPGKGLEWVAQIRNKPYNYETYYSNSVKG RFTISRDDAKSSVYLQMSSLRAEDTAVYYCTWAMI TSDGYAMDYWGQGTLVTVSS | 81 |
| VH5 (MP1-H37-V2) | EVQLVESGGGLVQPGGSLRLSCAASGITFSSYWM NWVRQAPGKGLEWVAQIKQKPYNYEKYYVQSVK GRFTISRDDAKSSLYLQMSSLRAEDTAVYYCAWAM ITSDGYAMDYWGQGTLVTVSS | 82 |
| VH6 | EVQLVESGGGLVQPGGSLRLSCAASGFTFSSYWM | 83 |
| (MP1-H37-V3) | SWVRQAPGKGLEWVAQIKQKPYGGEKYYVQSVK GRFTISRDDAKSSLYLQMSSLRAEDTAVYYCAWAM ITSDGYAFDYWGQGTLVTVSS | |
| VH7 (MP1-H333-V1) | QVKVVESGGGVVQPGRSLRLSCAASGITFSDYWM NWVRQAPGKGLEWVAQIRNKPYNYETYYSNSVKG RFTISRDDSKSTVYLQMSSLRAEDTAVYYCTWAMI TSDGYAMDYWGQGTLVTVSS | 84 |
| VH8 (MP1-H333-V2) | QVQLVESGGGVVQPGRSLRLSCAASGITFSSYWM NWVRQAPGKGLEWVAQIRNKPYNYEKYYAQSVK GRFTISRDDSKSTLYLQMSSLRAEDTAVYYCAWAM ITSDGYAMDYWGQGTLVTVSS | 85 |
| VH9 (MP1-H333-V3) | QVQLVESGGGVVQPGRSLRLSCAASGFTFSSYW MHWVRQAPGKGLEWVAVIRYKPYGSEKYYAQSVK GRFTISRDDSKSTLYLQMSSLRAEDTAVYYCAWAM ITSDGYAFDYWGQGTLVTVSS | 86 |

| Light chain variable region | Amino acid sequence | SEQ ID NO: |
|---|---|---|
| VL1 (MP1-L41-V1) | DIVMTQSPDSLAVSLGERATISCKSSQSLLLSSNQK NYLAWYQQKPGQPPKLLIYWASTRESGVPDRFSG SGSGTEFTLTISSVQAEDVAVYYCQQCFSYPLTFG QGTKLEIK | 88 |
| VL2 (MP1-L41-V2) | DIVMTQSPDSLAVSLGERATISCKSSQSVLLSSNQK NYLAWYQQKPGQPPKLLIYWASTRESGVPDRFSG SGSGTEFTLTISSLQAEDVAVYYCQQSFSYPLTFGQ GTKLEIK | 89 |
| VL3 (MP1-L41-V3) | DIVMTQSPDSLAVSLGERATISCKSSQSVLYSSNQK NYLAWYQQKPGQPPKLLIYWASTRESGVPDRFSG SGSGTEFTLTISSLQAEDVAVYYCQQSFSTPYTFG QGTKLEIK | 90 |
| VL4 (MP1-L320-V1) | EIVMTQSPGTLSLSPGERATLSCRSSQSLLLSSNQ KNYLAWYQQKPGQAPRLLIYWASTRESGIPDRFS GSGSGTDFTLTISRVEPEDFAVYYCQQCFSYPLTF GQGTKLEIK | 91 |
| VL5 (MP1-L320-V2) | EIVLTQSPGTLSLSPGERATLSCRASQSVLLSSNQK NYLAWYQQKPGQAPRLLIYWASSRETGIPDRFSG SGSGTDFTLTISRLEPEDFAVYYCQQSFSYPLTFGQ GTKLEIK | 92 |
| VL6 (MP1-L320-V3) | EIVLTQSPGTLSLSPGERATLSCRASQSVLLSSSQK NYLAWYQQKPGQAPRLLIYWASSRATGIPDRFSGS GSGTDFTLTISRLEPEDFAVYYCQQSFSSPYTFGQ GTKLEIK | 93 |
| VL7 (MP1-L228-V1) | DIVMTQSPLSLPVTPGEPASISCRSSQSLLLSSNQK NYLAWYLQKPGQSPQLLIYWASTRESGVPDRFSG SGSGTDFTLKISRVEAEDVGVYYCQQCFSYPLTFG QGTKLEIK | 94 |
| VL8 (MP1-L228-V2) | DIVMTQSPLSLPVTPGEPASISCRSSQSLLLSSNQK NYLAWYLQKPGQSPQLLIYWGSTRESGVPDRFSG SGSGTDFTLKISRVEAEDVGVYYCQQSFSYPLTFG | 95 |
| | QGTKLEIK | |
| VL9 (MP1-L228-V3) | DIVMTQSPLSLPVTPGEPASISCRSSQSLLHSSNGY NYLAWYLQKPGQSPQLLIYWGSTRASGVPDRFSG SGSGTDFTLKISRVEAEDVGVYYCQQSFQTPYTFG QGTKLEIK | 96 |

### Example 7.2 Expression and analysis of humanized recombinant antibodies

The amino acid sequences corresponding to expression genes of the 9 kinds of heavy chains (including VH1 to VH9) and he 9 kinds of light chains (VL1 to VL9) of Table 14 above were codon-optimized to synthesize DNA sequences, and using the synthesized DNA, they were connected to human Fc region genes (human IgG1 Fc gene for the heavy chains, human Ig kappa constant gene for the light chains) by overlap PCR, to clone them into pcDNA3.4 vector comprised in ExpiCHO-S Expression system kit (Gibco, USA). Plasmids expressing the light chains and heavy chains of the manufactured humanized MP1 antibody were transfected into ExpiCHO-S cell line using ExpiCHO-S expression system kit (Invitrogen/Life technologies) to produce transient expression in a 48-well scale.

Using the expressed humanized antibody culture solution, 1) the presence or absence of cell surface binding by staining the pancreatic cancer cell line Capan-2, and 2) the presence or absence of the humanized antibody expression through antihuman Ig antibody sandwich ELISA were confirmed. Among a total of 81 kinds of heavy chain and light chain combinations (namely, antibody combinations comprising a heavy chain comprising any one heavy chain variable region selected from the 9 kinds of heavy chain variable regions described in Table 14 and a light chain comprising any one light chain variable region selected from the 9 kinds of light chain variable regions described in Table 14, a humanized antibody combination showing a similar pattern of binding to the cell surface of the original mouse antibody or chimeric antibody could be confirmed, and it could be confirmed that the antibodies were expressed overall based on the sandwich ELISA result that confirmed the expression level of the transiently expressed humanized antibody (Table 15).

**[Table 15]**

| Antibody (VH#/VL#) | Capan-2 cell surface binding (%) | expression (sandwich ELISA) | Antibody (VH#/VL#) | Capan-2 cell surface binding (%) | expression (sandwich ELISA) |
|---|---|---|---|---|---|
| VH8/VL3 | 28.5 | 1.6380 | VH4/VL8 | 11.4 | 0.9107 |
| VH1/VL5 | 27.2 | 1.7730 | VH4/VL7 | 11 | 0.9468 |
| VH4/VL4 | 18.1 | 1.5129 | VH7/VL5 | 10.2 | 1.5669 |
| VH4/VL5 | 16.9 | 2.1846 | VH8/VL6 | 9.97 | 2.3048 |
| VH1/VL4 | 15.8 | 1.1142 | VH1/VL1 | 7.95 | 0.7590 |
| VH8/VL4 | 15.6 | 0.6951 | VH4/VL2 | 7.2 | 1.8891 |
| VH1/VL2 | 13.5 | 1.3022 | VH8/VL8 | 7.16 | 0.7846 |
| VH7/VL4 | 12.7 | 1.0518 | VH9/VL2 | 7.04 | 1.2997 |
| VH7/VL3 | 11.5 | 1.8322 | VH8/VL1 | 6.65 | 1.5327 |
| VH8/VL2 | 11.4 | 2.1098 | VH8/VL5 | 6.61 | 0.6999 |

In Table 15 above, cell surface binding (%) represents the positive rate of the antibody binding to the cell surface compared to the negative control group as %, and expression (sandwich ELISA) is a 450 nm OD value of sandwich ELISA.

Among the manufactured 81 kinds of antibodies in Table 15 above, 20 antibodies with high Capan-2 cell surface binding (%) were shown. As could be confirmed in Table 12, VH8/VL3 (an antibody comprising a heavy chain comprising the heavy chain variable region VH8 of Table 14 and a light chain comprising the light chain variable region VL3 of Table 14; hereinafter, same), VH1/VL5, VH4/VL4, VH4/VL5, VH1/VL4, VH8/VL4, VH1/VL2, VH7/VL4, VH7/VL3, VH8/VL2, VH4/VL8, and VH4/VL7 antibodies showed excellent Capan-2 cell surface binding.

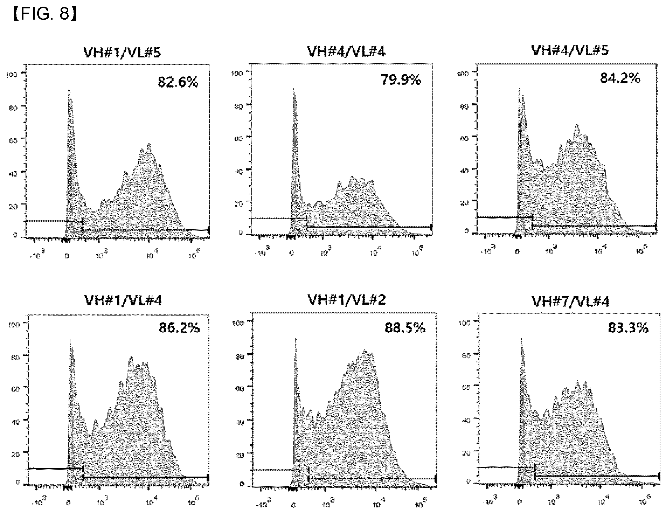

The antibody gene of the humanized antibody heavy chain and light chain combination with high positivity in the primary transient expression was expanded from the 48-well to 6-well scale and thereby, transient expression was performed again, and then the binding capacity to the Capan-2 pancreatic cancer cell line was reconfirmed (FIG. 8).

As FIG. 8, in the VH1/VL5, VH4/VL4, VH4/VL5, VH1/VL4, VH1/VL2, and VH7/VL4 antibodies, Capan-2 cell surface binding was excellent.

### Example 8. in vitro efficacy: T-cell engaging bispecific Ab / format optimization

### Example 8-1. Expression of T cell engaging bispecific antibody using humanized MP1 antibody

Among various bispecific antibody forms, a tetravalent bispecific antibody which showed T cell activity and high anticancer function, and possessed an Fc region at the same time, and therefore, maintained a half-life closely to that of the original antibody was expressed. The anti-CD3 antibody for T cell activity used the humanized antibody variable region of OKT3 antibody showing T cell activity. The sequence of the humanized OKT3 antibody was cited and used from US 20090202532 A1 patent, and it is the sequence corresponding to SEQ ID NO: 103, SEQ ID NO: 105 and SEQ ID NO: 106 in Table 13 below. SEQ ID NO: 103 is the amino acid sequence of the heavy chain variable region of the humanized OKT3 antibody, and SEQ ID NO: 104 is the amino acid sequence of the light chain variable region of the humanized OKT3 antibody, and SEQ ID NO: 105 represents the amino acid sequence of the scFv antibody of the humanized OKT3 antibody. The humanized OKT3 antibody was composed of a single chain Fv (scFv) connected with [GGGS]n=5 linker between the heavy chain variable region sequence and light chain variable region sequence, and it was to be connected to the light chain N-terminus (LCN version) or C-terminus (LCC version) of the humanized MP1 antibody with [GGGS]n=3 linker.

For the humanized OKT3 scFv, in order to increase stability by inducing interdomain disulfide bonds, No. 44 glycine of the heavy chain variable region and No. 100 glycine of the light chain variable region were replaced with cysteine (Protein Engineering, Design & Selection vol. 25 no. 7 pp. 321- 329, 2012).

To express it as a bispecific antibody, a humanized MP1 heavy chain gene (gene encoding any one heavy chain of VH1 to VH9 in Table 11 above) plasmid and an LCN or LCC type of humanized MP1 light chain-OKT3scFv gene (gene encoding the light chain sequence comprising light chain complementarity determining regions of VL4 or VL5 in Table 11 above - OKT3scFv gene) plasmid were transfected into ExpiCHO-S cells at a ratio of 1 : 1.7. After 7 days or 10 days, using the transfected bispecific antibody culture solution, antibody expression (sandwich ELISA using antihuman Ig), cell line binding (cell surface staining and flow cytometer analysis), and cytotoxicity assay were performed, and the bispecific antibody culture solution of which activity was confirmed was purified by Protein A affinity chromatography method.

The sequence of the anti-MUC1/anti-CD3 bispecific antibody manufactured above (light chain sequence of the anti-MUC1 antibody and scFv sequence of the anti-CD3 antibody) was shown in Table 13 below. The form in which the anti-CD3 antibody (OFT3 scFv) was bound to the C-terminus of the light chain of the anti-MUC1 antibody was denoted as LCC, and the form in which it was bound to the N-terminus of the light chain was denoted as LCN.

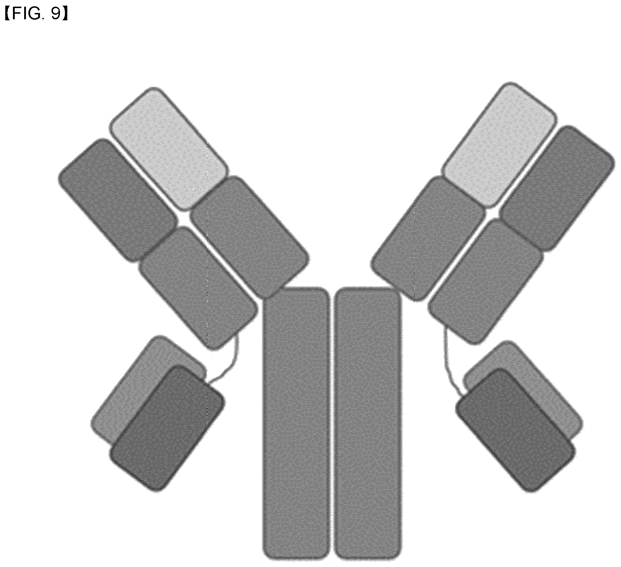

In Table 16 below, the LCC type of the anti-MUC1/anti-CD3 bispecific antibody (See FIG. 9), LCC version humanized MP1 Light chain #4-OKT3scFv represents the light chain sequence (SEQ ID NO: 112) of the anti-MUC1/anti-CD3 bispecific antibody in which the humanized OKT3 scFv antibody (SEQ ID NO: 106) was bound to the C-terminus of the light chain sequence (SEQ ID NO: 110) comprising the light chain variable region (SEQ ID NO: 91) of light chain No. 4 (VL4) of Table 14. LCC version humanized MP1 Light chain #5-OKT3scFv represents the light chain sequence (SEQ ID NO: 113) of the anti-MUC1/anti-CD3 bispecific antibody in which the humanized OKT3 scFv antibody (SEQ ID NO: 106) was bound to the C-terminus of the light chain sequence (SEQ ID NO: 111) comprising the light chain variable region (SEQ ID NO: 92) of light chain No. 5 (VL5) of Table 14.

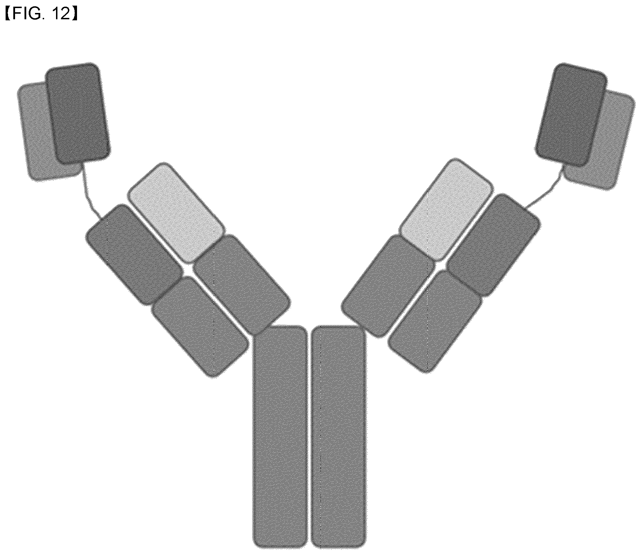

In Table 16 below, the LCN type of anti-MUC1/anti-CD3 bispecific antibody (See FIG. 12), LCN version humanized MP1 Light chain #4-OKT3scFv represents the light chain sequence (SEQ ID NO: 114) of the anti-MUC1/anti-CD3 bispecific antibody in which the humanized OKT3 scFv antibody (SEQ ID NO: 106) was bound to the N-terminus of the light chain sequence (SEQ ID NO: 110) comprising the light chain variable region (SEQ ID NO: 91) of light chain No. 4 (VL4) of Table 14. LCN version humanized MP1 Light chain #4-OKT3scFv represents the light chain sequence (SEQ ID NO: 115) of the anti-MUC1/anti-CD3 bispecific antibody in which the OKT3 scFv antibody (SEQ ID NO: 106) was bound to the C-terminus of the light chain sequence (SEQ ID NO: 111) comprising the light chain variable region (SEQ ID NO: 92) of light chain No. 5 (VL5) of Table 14.

In the 4 kinds of light chain sequences of the anti-MUC1/anti-CD3 bispecific antibody, the light chain of the anti-MUC1 antibody and the humanized OKT3 scFv antibody were linked with [GGGS]n=3 linker. As the heavy chain of the anti-MUC1/anti-CD3 bispecific antibody, a heavy chain sequence comprising the heavy chain variable regions (SEQ ID NO: 78 to SEQ ID NO: 86) of heavy chain No. 1 to heavy chain No. 9 of Table 14 was used.

**[Table 16]**

| CD3 antibody used for bispecific antibody expression and light chain of bispecific antibody using the same | Sequence | SEQ ID NO: |
|---|---|---|
| humanized OKT3 CD3Ab VH | QVQLVQSGGGVVQPGRSLRLSCKASGYTFTRYT MHWVRQAPGKGLEWIGYINPSRGYTNYNQKVKD RFTISRDNSKNTAFLQMDSLRPEDTGVYFCARYY DDHYSLDYWGQGTPVTVSS | 103 |
| humanized OKT3 CD3Ab VL | DIQMTQSPSSLSASVGDRVTITCSASSSVSYMNW YQQTPGKAPKRWIYDTSKLASGVPSRFSGSGSG TDYTFTISSLQPEDIATYYCQQWSSNPFTFGQGT KLQITR | 105 |
| humanized OKT3 scFv | QVQLVQSGGGVVQPGRSLRLSCKASGYTFTRYT MHWVRQAPGKCLEWIGYINPSRGYTNYNQKVKD RFTISRDNSKNTAFLQMDSLRPEDTGVYFCARYY DDHYSLDYWGQGTPVTVSSGGGSGGGSGGGS GGGSGGGSDIQMTQSPSSLSASVGDRVTITCSA SSSVSYMNWYQQTPGKAPKRWIYDTSKLASGVP SRFSGSGSGTDYTFTISSLQPEDIATYYCQQWSS NPFTFGQCTKLQITR | 106 |
| VL4 light chain | EIVMTQSPGTLSLSPGERATLSCRSSQSLLLSSN QKNYLAWYQQKPGQAPRLLIYWASTRESGIPDR FSGSGSGTDFTLTISRVEPEDFAVYYCQQCFSYP LTFGQGTKLEIKRTVAAPSVFIFPPSDEQLKSGTA SVVCLLNNFYPREAKVQWKVDNALQSGNSQESV TEQDSKDSTYSLSSTLTLSKADYEKHKVYACEVT HQGLSSPVTKSFNRGEC | 110 |
| VL5 light chain | EIVLTQSPGTLSLSPGERATLSCRASQSVLLSSNQ KNYLAWYQQKPGQAPRLLIYWASSRETGIPDRFS GSGSGTDFTLTISRLEPEDFAVYYCQQSFSYPLTF GQGTKLEIKRTVAAPSVFIFPPSDEQLKSGTASVV CLLNNFYPREAKVQWKVDNALQSGNSQESVTEQ DSKDSTYSLSSTLTLSKADYEKHKVYACEVTHQG LSSPVTKSFNRGEC | 111 |
| LCC version humanized MP1 Light chain #4-OKT3scFv | EIVMTQSPGTLSLSPGERATLSCRSSQSLLLSSN QKNYLAWYQQKPGQAPRLLIYWASTRESGIPDR FSGSGSGTDFTLTISRVEPEDFAVYYCQQCFSYP LTFGQGTKLEIKRTVAAPSVFIFPPSDEQLKSGTA SVVCLLNNFYPREAKVQWKVDNALQSGNSQESV TEQDSKDSTYSLSSTLTLSKADYEKHKVYACEVT HQGLSSPVTKSFNRGECGGGSGGGSGGGSQV QLVQSGGGVVQPGRSLRLSCKASGYTFTRYTMH WVRQAPGKCLEWIGYINPSRGYTNYNQKVKDRF TISRDNSKNTAFLQMDSLRPEDTGVYFCARYYDD | 112 |
| | HYSLDYWGQGTPVTVSSGGGSGGGSGGGSGG GSGGGSDIQMTQSPSSLSASVGDRVTITCSASSS VSYMNWYQQTPGKAPKRWIYDTSKLASGVPSRF SGSGSGTDYTFTISSLQPEDIATYYCQQWSSNPF TFGQCTKLQITR | |
| LCC version humanized MP1 Light chain #5-OKT3scFv | EIVLTQSPGTLSLSPGERATLSCRASQSVLLSSNQ KNYLAWYQQKPGQAPRLLIYWASSRETGIPDRFS GSGSGTDFTLTISRLEPEDFAVYYCQQSFSYPLTF GQGTKLEIKRTVAAPSVFIFPPSDEQLKSGTASVV CLLNNFYPREAKVQWKVDNALQSGNSQESVTEQ DSKDSTYSLSSTLTLSKADYEKHKVYACEVTHQG LSSPVTKSFNRGECGGGSGGGSGGGSQVQLVQ SGGGVVQPGRSLRLSCKASGYTFTRYTMHWVR QAPGKCLEWIGYINPSRGYTNYNQKVKDRFTISR DNSKNTAFLQMDSLRPEDTGVYFCARYYDDHYS LDYWGQGTPVTVSSGGGSGGGSGGGSGGGSG GGSDIQMTQSPSSLSASVGDRVTITCSASSSVSY MNWYQQTPGKAPKRWIYDTSKLASGVPSRFSGS GSGTDYTFTISSLQPEDIATYYCQQWSSNPFTFG QCTKLQITR | 113 |
| LCN version humanized MP1 Light chain #4-OKT3scFv | QVQLVQSGGGVVQPGRSLRLSCKASGYTFTRYT MHWVRQAPGKCLEWIGYINPSRGYTNYNQKVKD RFTISRDNSKNTAFLQMDSLRPEDTGVYFCARYY DDHYSLDYWGQGTPVTVSSGGGSGGGSGGGS GGGSGGGSDIQMTQSPSSLSASVGDRVTITCSA SSSVSYMNWYQQTPGKAPKRWIYDTSKLASGVP SRFSGSGSGTDYTFTISSLQPEDIATYYCQQWSS NPFTFGQCTKLQITRGGGSGGGSGGGSEIVMTQ SPGTLSLSPGERATLSCRSSQSLLLSSNQKNYLA WYQQKPGQAPRLLIYWASTRESGIPDRFSGSGS GTDFTLTISRVEPEDFAVYYCQQCFSYPLTFGQG TKLEIKRTVAAPSVFIFPPSDEQLKSGTASVVCLLN NFYPREAKVQWKVDNALQSGNSQESVTEQDSK DSTYSLSSTLTLSKADYEKHKVYACEVTHQGLSS PVTKSFNRGEC | 114 |
| LCN version humanized MP1 Light chain #5-OKT3scFv | QVQLVQSGGGVVQPGRSLRLSCKASGYTFTRYT MHWVRQAPGKCLEWIGYINPSRGYTNYNQKVKD RFTISRDNSKNTAFLQMDSLRPEDTGVYFCARYY DDHYSLDYWGQGTPVTVSSGGGSGGGSGGGS GGGSGGGSDIQMTQSPSSLSASVGDRVTITCSA SSSVSYMNWYQQTPGKAPKRWIYDTSKLASGVP SRFSGSGSGTDYTFTISSLQPEDIATYYCQQWSS NPFTFGQCTKLQITRGGGSGGGSGGGSEIVLTQ SPGTLSLSPGERATLSCRASQSVLLSSNQKNYLA WYQQKPGQAPRLLIYWASSRETGIPDRFSGSGS GTDFTLTISRLEPEDFAVYYCQQSFSYPLTFGQGT KLEIKRTVAAPSVFIFPPSDEQLKSGTASVVCLLN | 115 |
| | NFYPREAKVQWKVDNALQSGNSQESVTEQDSK DSTYSLSSTLTLSKADYEKHKVYACEVTHQGLSS PVTKSFNRGEC | |

### Example 8-2. Analysis of function of LCC type humanized MP1 bispecific antibody

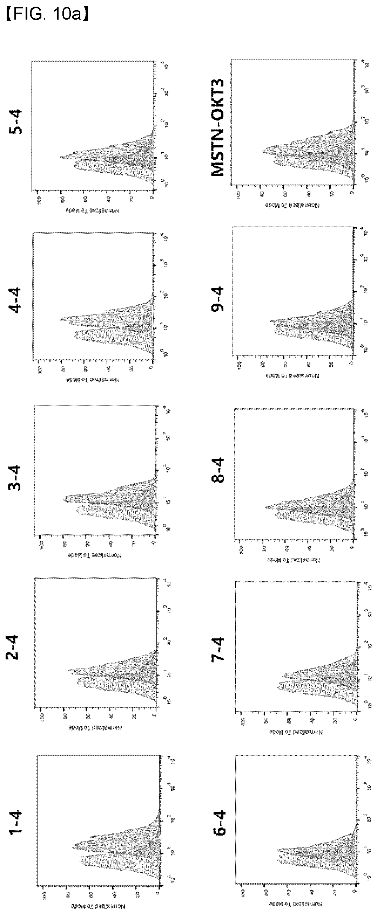

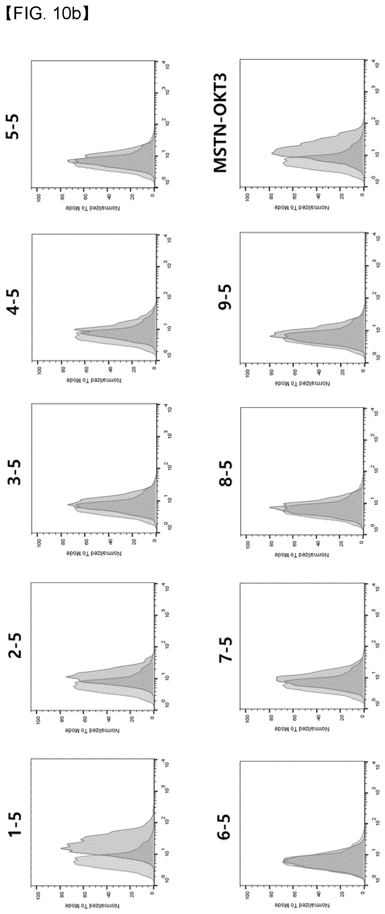

For the LCC type of anti-MUC1/anti-CD3 bispecific antibody prepared in Example 8-1, surface binding affinity and cytotoxicity assay were performed on AsPC1 pancreatic cancer cells positive to the MUC1 antigen. It was confirmed that the anti-MUC1/anti-CD3 bispecific antibody was expressed at a protein level by Sandwich ELISA using anti-human Ig/anti-human Ig-HRP (Table 17), and it was confirmed that these bispecific antibodies bound to the pancreatic cancer cell positive to the MUC1 antigen, AsPC1 by cell surface staining and flow cytometry (FIG. 10).

As a control group, the anti-mesothelin/OKTscFv humanized bispecific antibody (MSTN-OKT3) was used. The MSTN-OKT3 is an LCC type of bispecific antibody in which the humanized OKT3 scFv sequence (SEQ ID NO: 106) was bound to the C-terminus of the light chain of the h7D9.v3 antibody, using the sequence of h7D9.v3 antibody described in International laid-open publication (WO 2012/087962).

**[Table 17]**

| Antibody | Expression | Antibody | Expression |
|---|---|---|---|
| VH1/VL5 | 2.4501 | VH1/VL4 | 1.8550 |
| VH2/VL5 | 1.4389 | VH2/VL4 | 1.2238 |
| VH3/VL5 | 1.5525 | VH3/VL4 | 1.2721 |
| VH4/VL5 | 2.2260 | VH4/VL4 | 1.5691 |
| VH5/VL5 | 1.2155 | VH5/VL4 | 0.7501 |
| VH6/VL5 | 1.3825 | VH6/VL4 | 0.9205 |
| VH7/VL5 | 2.1425 | VH7/VL4 | 1.5371 |
| VH8/VL5 | 1.7722 | VH8/VL4 | 1.6626 |
| VH9/VL5 | 1.2984 | VH9/VL4 | 1.0687 |

In Table 17 above, expression (sandwich ELISA) is a 450 nm OD value of sandwich ELISA, and in one embodiment, the antibody "VH1/VL5" means the anti-MUC1/anti-CD3 bispecific antibody comprising a heavy chain comprising the heavy chain variable region (SEQ ID NO: 78) of heavy chain No. 1 (VH1) of Table 14 and a heavy chain (SEQ ID NO: 113) in which the humanized OKT3 scFv antibody (SEQ ID NO: 106) was bound to the C-terminus of the light chain variable region (SEQ ID NO: 92) of light chain No. 5 (VL5) of Table 14.

That they showed negativity in flow cytometry in FIG. 10, although in Table 17 above, the antibodies were expressed, is because the affinity of the original antibody was lost in the humanized antibody combination process. In addition, as could be confirmed in FIG. 10, among the bispecific antibody combinations, the bispecific antibody combinations expressing light chain No. 4-OKT3 scFv antibody together (i.e., bispecific antibody comprising any one heavy chain variable region among heavy chain No. 1 to heavy chain No. 9 of Table 14 and a light chain the humanized OKT3 scFv antibody was bound to the C-terminus of the light chain variable region of light chain No. 4) showed cell surface binding all, but among the bispecific antibody combinations expressing light chain No. 5-OKT3 scFv antibody together, only the combinations of heavy chain No. 1/light chain No. 5, heavy chain No. 2/light chain No. 5, and heavy chain No. 7/light chain No. 5 showed cell surface binding.

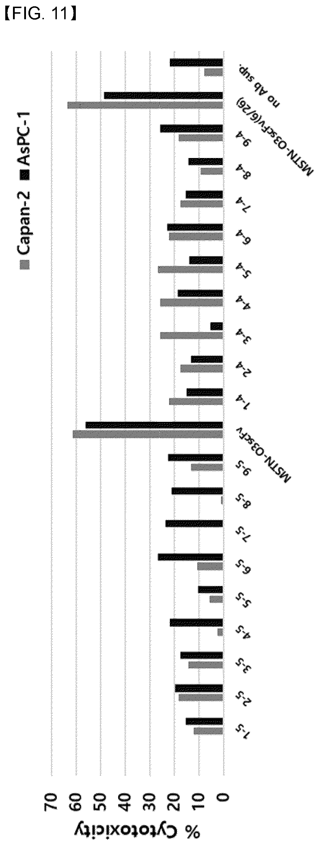

For the expressed bispecific antibodies, using Capan-2 and AsPC-1 cell lines as target cells, and human T cells as effector cells, cytotoxicity assay was performed. The ratio of the target cells and effector cells was set to 1:10, and the input amount of the bispecific antibody was same at 1 ug/ml. After 48 hours, the death of the target cells was measured using EZ-Cytox (DogenBio, Cat.#: EZ-1000), and % cytotoxicity was calculated using the following formula, and this was shown in FIG. 11. % Cytotoxicity = (1-(antibody administration well measurement value/target and effector cell well measurement value))X100

As could be confirmed in FIG. 11, the effect was not well shown, as the LCC type of anti-MUC1/anti-CD3 bispecific antibody prepared in Example 8-2 had a lower effect than the control antibody (MSTN-OKT3), and the cytotoxicity was less than 30%.

### Example 8-3. Analysis of functions of LCN type humanized MP1 bispecific antibody (1)

For the LCN type of anti-MUC1/anti-CD3 bispecific antibody prepared in Example 8-1, the Capan-1, AsPC1 pancreatic cancer cell surface binding capacity and cytotoxicity assay, which are positive for the MUC1 antigen, were performed.

It was confirmed that the LCN type of anti-MUC1/anti-CD3 bispecific antibody expressed as Table 16 above was expressed at a protein level by Sandwich ELISA using anti-human Ig/anti-human Ig-HRP, and it was confirmed that these bispecific antibodies bound to Capan-1 and AsPC1, which are pancreatic cancer cells that are positive for the MUC1 antigen, by staining and flow cytometry.

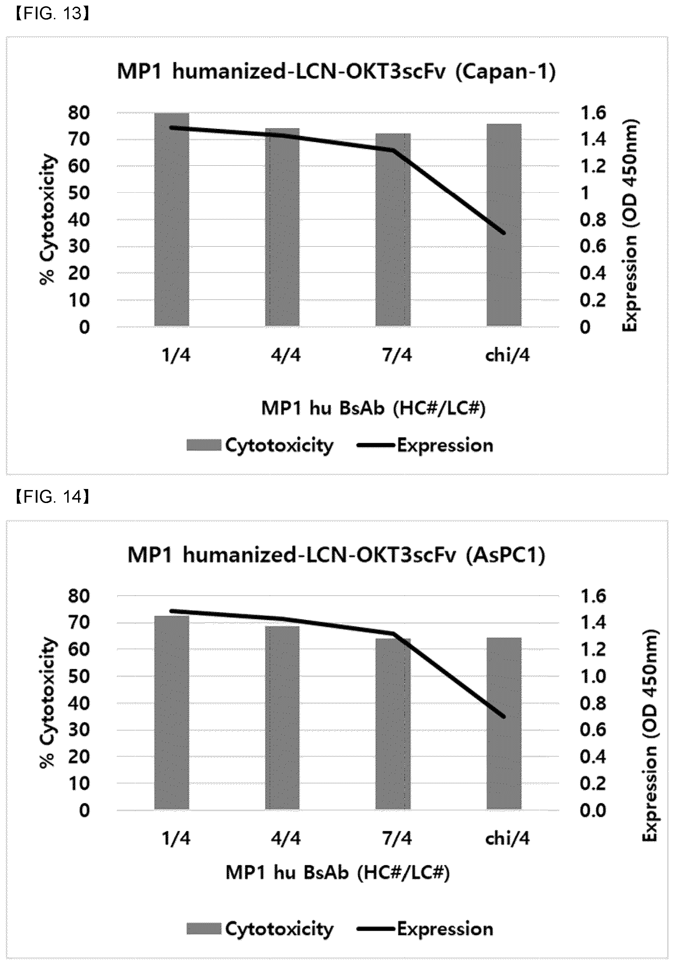

Such a result was shown in FIG. 13 and FIG. 14. As a control group, the anti-MUC1/anti-CD3 bispecific antibody (chi/4) comprising the heavy chain comprising the heavy chain variable region (SEQ ID NO: 77) of the mouse MUC1 antibody of Table 12 and the light chain in which the humanized OKT3 scFv antibody was combined to the N-terminus of the light chain sequence (SEQ ID NO: 110) comprising the light chain variable region (SEQ ID NO: 91) of the light chain No. 4 (VL4) of Table 14 was used.

As shown in FIG. 13 and FIG. 14, it was confirmed that the anti-MUC1/anti-CD3 bispecific antibodies of the combination of heavy chain No. 1/light chain No. 4 ("1/4", that is, the anti-MUC1/anti-CD3 bispecific antibody comprising the heavy chain comprising the heavy chain variable region (SEQ ID NO: 78) of heavy chain No. 1 (VH1) of Table 14 and the light chain (SEQ ID NO: 114) in which the humanized OKT3 scFv antibody (SEQ ID NO: 106) was combined to the N-terminus of the light chain variable region (SEQ ID NO: 91) of light chain No. 4 (VL4) of Table 14; same hereinafter.), heavy chain No.4/light chain No. 4 ("4/4"), heavy chain No. 7/light chain No. 4 ("7/4") had an excellent toxic effect against cancer cells compared to the control group.

This result shows that the LCN type of bispecific antibody (Example 8-3) can bind by maintaining a closer microdistance between cancer cells and T cells, which are easy to exhibit cytotoxicity than the LCC type of bispecific antibody (Example 8-2), and through this, it can show further higher cytotoxicity.

### Example 8-4. Analysis of functions of LCN type humanized MP1 bispecific antibody (2)

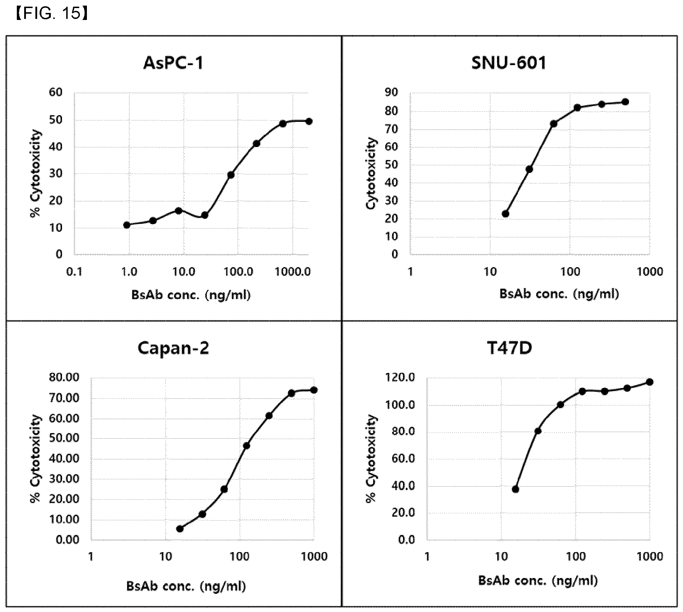

For the LCN type of the anti-MUC1/anti-CD3 bispecific antibody prepared in Example 8-1, for the AsPC-1 and Capan-2 (pancreatic cancer cell line), SNU-601 (gastric cancer cell line), and T47D (breast cancer cell line) cell lines positive for the MUC1 antigen, cytotoxicity assay was performed, and the in vitro cytotoxic effect was confirmed, and the result was shown in FIG. 15.

As the anti-MUC1/anti-CD3 bispecific antibody, the anti-MUC1/anti-CD3 bispecific antibody of the heavy chain No. 1/light chain No. 5 with a higher humanization ratio, that is, the anti-MUC1/anti-CD3 bispecific antibody comprising the heavy chain comprising the heavy chain variable region (SEQ ID NO: 78) of the heavy chain No. 1 (VH1) of Table 14 and the light chain (SEQ ID NO: 115) in which the humanized OKT3 scFv antibody (SEQ ID NO: 106) was combined to the N-terminus of the light chain variable region (SEQ ID NO: 92) of the light chain No. 5 (VL5) of Table 14, was used.

As shown in FIG. 15, it was confirmed that a cytotoxic effect against cancer cells was shown in proportion to the concentration of the administered LCN type of anti-MUC1/anti-CD3 bispecific antibody.

### Example 9. Evaluation of treatment effect of humanized bispecific antibody using pancreatic cancer mouse experimental model

In order to confirm the in vivo immune-anticancer effect of the anti-MUC1/anti-CD3 bispecific antibody, for the NOG mouse animal model in which a human body-derived pancreatic cancer cell line, AsPC-1 was transplanted, whether the size of the tumor was reduced by administration of the anti-MUC1/anti-CD3 bispecific antibody was evaluated. As the anti-MUC1/anti-CD3 bispecific antibody, the same antibody as the antibody used in Example 8-4 was used (anti-MUC1/anti-CD3 bispecific antibody of the combination of the LCN type heavy chain No. 1/light chain No. 5).

### Mouse model preparation

In order to evaluate the anticancer effect of the antibody by substantial human immunocytes, as the mice, NOG mice (NOD/Shi-scid, IL-2RγKO mice, Koatech), which were deficient in functions of T cells, B cells, NK cells and macrophages, and dendritic cells, were used. The pancreatic cancer cell line, AsPC-1 of 5×10⁶ was subcutaneously injected (I.P., intraperitoneal injection) into the flanks of 8-week-old NOG mice, and after 1-2 weeks, 5 mice in each group, which were mice of which tumor volume of 100 ~ 150 mm³ was formed, were selected and used for an experiment.

### Human PBMC preparation

Human Cryopreserved Peripheral Blood Mononuclear Cell (PBMC) purchased from Zenbio company was thawed with RPMI1640 (GeneDireX,Cat#:CC112-0500) comprising 10% FBS 10 days before antibody injection, and then on day 1, IFN-r (jw creagene, Cat#: HIFNG-100) 250 ng/ml was treated and on day 2, IL-2 (jw creagene, JW-H031-0100) 30 ng/ml, OKT3 antibody (Miltenyi Biotec, Cat#: 130-093-387) 50 ng/ml were added and then left for 5 days. After that, the number of the PBMC was expanded by culturing IL-2 30 ng/ml, IFN-r 50 ng/ml in RPMI1640 medium ( in which 10% FBS was added from day 6 of culture to day 16 of culture.

### Evaluation of anticancer efficacy of humanized MP1 bispecific antibody

NOG mice with tumor formation were divided into six groups and on day 0, PBS 200 µ l was intraperitoneally injected into one group, and PBMC of the number of cells of 5×10⁶ per mouse was intraperitoneally injected into the 5 groups of mice (In other words, a suspension was prepared with 1XPBS so that it was to be 5×10⁶ cells/0.2 mL and intraperitoneally injected). After that, the anti-MUC1/anti-CD3 bispecific antibody was injected with PBMC into the 4 groups of mice at 1 mg/kg, 3 mg/kg, respectively, and after a total of 4 intravenous injections (I.V.) on days 3, 7, 10 and 14, the size of the cancer cells injected subcutaneously was observed and measured.

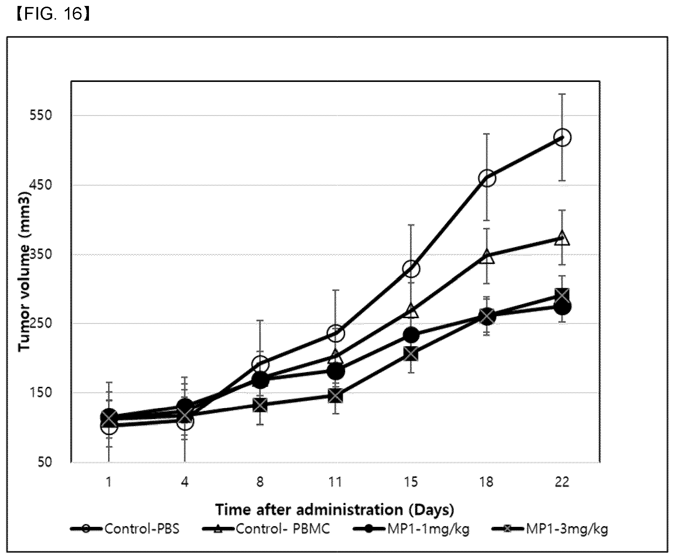

FIG. 16 is the result showing in vivo anticancer efficacy in the mouse pancreatic cancer animal model under the humanization condition using the anti-MUC1/anti-CD3 bispecific antibody in which PBMC was injected. The antibody administration group (MP1-1mg/kg and MP1-3mg/kg) showed a higher anticancer effect (Tumor growth inhibition; TGI) compared to the only PBMC administration group (Control-PBMC).

From the above description, those skilled in the art to which the present invention pertains will understand that the present invention can be implemented in other specific forms without changing its technical spirit or essential features. In this regard, the examples described above should be understood as illustrative and not restrictive in all aspects. The scope of the present invention should be interpreted as all changed or modified forms derived from the meaning and scope of the claims described below and equivalent concepts thereof are included in the scope of the present invention.

## Claims

1. An anti-MUC1 (Mucin-1) antibody or an antigen binding fragment thereof, comprising
a polypeptide having the amino acid sequence of SEQ ID NO: 1 (VH-CDR1),
a polypeptide having the amino acid sequence of SEQ ID NO: 7 (VH-CDR2),
a polypeptide having the amino acid sequence of SEQ ID NO: 16 (VH-CDR3),
a polypeptide having the amino acid sequence of SEQ ID NO: 19 (VL-CDR1),
a polypeptide having the amino acid sequence of SEQ ID NO: 27 (VL-CDR2), and
a polypeptide having the amino acid sequence of SEQ ID NO: 33 (VL-CDR3).

2. The anti-MUC1 antibody or antigen binding fragment thereof according to claim 1, comprising
a polypeptide having an amino acid sequence selected from the group consisting of SEQ ID NOs: 2 to 6 (VH-CDR1),
a polypeptide having an amino acid sequence selected from the group consisting of SEQ ID NOs: 8 to 15 (VH-CDR2),
a polypeptide having an amino acid sequence selected from the group consisting of SEQ ID NOs: 17 to 18 (VH-CDR3),
a polypeptide having an amino acid sequence selected from the group consisting of SEQ ID NOs: 20 to 26 (VL-CDR1),
a polypeptide having an amino acid sequence selected from the group consisting of SEQ ID NOs: 28 to 32 (VL-CDR2), and
a polypeptide having an amino acid sequence selected from the group consisting of SEQ ID NOs: 34 to 38 (VL-CDR3).

3. The anti-MUC1 antibody or antigen binding fragment thereof according to claim 1, comprising
a heavy chain variable region comprising an amino acid sequence selected from the group consisting of SEQ ID NOs: 77 to 86; and
a light chain variable region comprising an amino acid sequence selected from the group consisting of SEQ ID NOs: 87 to 96.

4. The anti-MUC1 antibody or antigen binding fragment thereof according to claim 1, wherein the anti-MUC1 antibody or antigen binding fragment has activity to induce apoptosis of cancer cells expressing MUC1.

5. The anti-MUC1 antibody or antigen binding fragment thereof according to claim 1, wherein the anti-MUC1 antibody is a monoclonal antibody.

6. The anti-MUC1 antibody or antigen binding fragment thereof according to claim 1, specifically recognizing a peptide comprising the amino acid sequence of SEQ ID NO: 109.

7. The anti-MUC1 antibody Biotic or antigen binding fragment thereof according to claim 1, wherein the antigen binding fragment is selected from the group consisting of scFv, (scFv)2, Fab, Fab' and F(ab')2 of the anti-MUC1 antibody.

8. The anti-MUC1 antibody or antigen binding fragment thereof according to claim 1, wherein the anti-MUC1 antibody is a mouse-derived antibody, a mouse-human chimeric antibody, a humanized antibody, or a human antibody.

9. A bispecific antibody, comprising the anti-MUC1 antibody or antigen binding fragment thereof of any one of claims 1 to 8, and at least one antigen binding site specific to a T cell activating antigen.

10. The bispecific antibody according to claim 9, wherein the T cell activating antigen is a CD3 T cell co-receptor (CD3) antigen.

11. The bispecific antibody according to claim 10, wherein the bispecific antibody comprises an anti-CD3 antibody or an antigen binding fragment thereof comprising an antigen binding site against a CD3 antigen,
wherein the anti-CD3 antibody or antigen binding fragment thereof is
an OKT3 antibody or an antigen binding fragment thereof, a UCHT-1 antibody or an antigen binding fragment thereof, or an SP34 antibody or an antigen binding fragment thereof.

12. The bispecific antibody according to claim 11, wherein the anti-CD3 antibody or antigen binding fragment thereof
is linked to the C-terminus or N-terminus of the anti-MUC1 antibody or antigen binding fragment thereof.

13. The bispecific antibody according to claim 11, wherein the antigen binding fragment of the anti-CD3 antibody is selected from the group consisting of scFv, (scFv)2, Fab, Fab' and F(ab')2 of the anti-CD3 antibody.

14. The bispecific antibody according to claim 11, wherein the anti-CD3 antibody or antigen binding fragment thereof comprises
a polypeptide having the amino acid sequence of SEQ ID NO: 97 (VH-CDR1),
a polypeptide having the amino acid sequence of SEQ ID NO: 98 (VH-CDR2),
a polypeptide having the amino acid sequence of SEQ ID NO: 99 (VH-CDR3),
a polypeptide having the amino acid sequence of SEQ ID NO: 100 (VL-CDR1),
a polypeptide having the amino acid sequence of SEQ ID NO: 101 (VL-CDR2), and
a polypeptide having the amino acid sequence of SEQ ID NO: 102 (VL-CDR3).

15. A pharmaceutical composition for prevention or treatment of cancer, comprising the anti-MUC1 antibody or antigen binding fragment thereof of any one of claims 1 to 8, or the bispecific antibody of any one of claims 9 to 14.

16. The pharmaceutical composition for prevention or treatment of cancer according to claim 15, wherein the cancer is at least one cancer selected from the group consisting of blood cancer, bone marrow cancer, thyroid cancer, cervical cancer, skin cancer, melanoma, breast cancer, colorectal cancer, lung cancer, gastric cancer, prostate cancer, and pancreatic cancer.

17. A polynucleotide encoding the anti-MUC1 antibody or antigen binding fragment thereof of any one of claims 1 to 8, or the bispecific antibody of any one of claims 9 to 14.

18. A recombinant vector comprising the polynucleotide of claim 17.

19. A recombinant cell, comprising the recombinant vector of claim 18.
